# EUROPEAN PATENT SPECIFICATION

(11) **EP 0 844 874 B1**
(45) Date of publication and mention of the grant of the patent: **09.11.2005**
(21) Application number: 96928868.7
(22) Date of filing: 13.08.1996
(51) Int. Cl.: A61K 31/12, A61K 38/00, A61K 38/04, A61P 27/02, C07K 5/00, C07K 7/00, C07K 16/00, C07K 17/00, C07K 14/78, C07C 311/10

(54) **METHODS AND COMPOSITIONS USEFUL FOR INHIBITION OF ALPHA v BETA 5 MEDIATED ANGIOGENESIS**
VERFAHREN UND MITTEL ZUR HEMMUNG DER DURCH ALPHA v BETA 5 VERMITTELTEN ANGIOGENESE
PROCEDE ET COMPOSITIONS UTILES POUR INHIBER L'ANGIOGENESE MEDIEE PAR ALPHA v BETA 5

(30) Priority: 14.08.1995 US 514799
(43) Date of publication of application: 03.06.1998
(73) Proprietor: THE SCRIPPS RESEARCH INSTITUTE, La Jolla, CA 92037 (US)
(72) Inventor: BROOKS, Peter, San Diego, CA 92122 (US); CHERESH, David, A., Cardiff, CA 92007 (US); FRIEDLANDER, Martin, Del Mar, CA 92014 (US)
(74) Representative: Fisher, Adrian John
(86) International application number: PCT/US1996/013194
(87) International publication number: WO 1997/006791

(56) References cited:
- EP-A- 0 578 083
- WO-A-95/14714
- WO-A-95/25543
- US-A- 5 235 919
- KLEIN S ET AL: "BASIC FIBROBLAST GROWTH FACTOR MODULATES INTEGRIN EXPRESSION IN MICROVASCULAR ENDOTHELIAL CELLS" MOLECULAR BIOLOGY OF THE CELL,US,BETHESDA, MD, vol. 4, no. 10, 1 October 1993 (1993-10-01), pages 973-982, XP000653405 ISSN: 1059-1524
- FRIEDLANDER M ET AL: "DEFINITION OF TWO ANGIOGENIC PATHWAYS BY DISTINCT ALPHAV INTEGRINS" SCIENCE,US,AMERICAN ASSOCIATION FOR THE ADVANCEMENT OF SCIENCE,, vol. 270, 1 December 1995 (1995-12-01), pages 1500-1502, XP000867841 ISSN: 0036-8075
- SCIENCE, 22 April 1994, Vol. 264, BROOKS et al., "Requirement of Vascular Integrin alphavbeta3 for Angiogenesis", pages 569-571, XP002138524.
- CELL, 03 April 1992, Vol. 69, HYNES, "Integrins: Versatility, Modulation and Signalling in Cell Adhesion", pages 11-25, XP000263014.
- CANCER RESEARCH, 15 April 1994, Vol. 54, LEHMANN et al., "A Monoclonal Antibody Inhibits Adhesion to Fibronectin and Vitronectin of a Colon Carcinoma Cell Line and Recognizes the Integrins alphavbeta3, alphavbeta5, and alphavbeta6", pages 2102-2107, XP002103619.
- FEBS LETTERS, October 1991, Vol. 291, AUMAILLEY et al., "Arg-Gly-Asp Constrained Within Cyclic Pentapeptides, Strong and Selective Inhibitors of Cell Adhesion to Vitronectin and Laminin Fragment P1", pages 50-54, XP000225814.
- JOURNAL OF BIOLOGICAL CHEMISTRY, 25 December 1987, Vol. 262, No. 36, PIERSCHBACHER et al., "Influence of Stereochemistry of the Sequence Arg-Gly-Asp-Xaa on Binding Specificity in Cell Adhesion", pages 17294-17298. XP001000941.
- JOURNAL OF BIOLOGICAL CHEMISTRY, 05 June 1992, Vol. 267, No. 16, FOLKMAN et al., "Angiogenesis", pages 10931-10934, XP000999214.
- SEMINARS IN CANCER BIOLOGY, 1992, Vol. 3, FOLKMAN et al., "Inhibition of Angiogenesis", pages 89-96, XP000604165.
- INT. J. CANCER, 1993, Vol. 55, HARDAN et al., "Inhibition of Metastatic Cell Colonization in Murine Lungs and Tumor-Induced Morbidity by Non-Peptidic Arg-Gly-Asp Mimetics", pages 1023-1028, XP000925214.
- FOLKMAN ET AL: 'ANGIOGENESIS IN CANCER, VASCULAR, RHEUMATOID AND OTHER DISEASE' NATURE MEDICINE, NATURE PUBLISHING, CO, US vol. 1, no. 1, 01 January 1995, USA, pages 27 - 31, XP000605147

### SEQUENCE LISTING

(1) GENERAL INFORMATION:
   (i) APPLICANT: THE SCRIPPS RESERCH INSTITUTE
   (ii) TITLE OF INVENTION: METHODS AND COMPOSITIONS USEFUL FOR INHIBITION OF ALPHA V BETA5-MEDIATED ANGIOGENESIS
   (iii) NUMBER OF SEQUENCES: 9
   (iv) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.25
   (v) CURRENT APPLICATION DATA:
      (A) APPLICATION NUMBER: PCT/US96/
      (B) FILING DATE: 13-AUG-1996
      (C) CLASSIFICATION:
   (vi) PRIOR APPLICATION DATA:
      (A) APPLICATION NUMBER: US 08/514,799
      (B) FILING DATE: 14-AUG-1995
(2) INFORMATION FOR SEQ ID NO:1:
   (i) SEQUENCE CHARACTERISTICS:
      (A) LENGTH: 5 amino acids
      (B) TYPE: amino acid
      (D) TOPOLOGY: linear
   (ii) MOLECULE TYPE: peptide
   (v) FRAGMENT TYPE: internal
   (ix) FEATURE:
      (A) NAME/KEY: Peptide
      (B) LOCATION: 1
      (D) OTHER INFORMATION: /label- BOC
   (ix) FEATURE:
      (A) NAME/KEY: Peptide
      (B) LOCATION: 4
      (D) OTHER INFORMATION: /label- D-Phe
         /note= "A prefix "D" in D-Phe signifies that the phenyalanine in position 4 is a D-amino acid."
   (ix) FEATURE:
      (A) NAME/KEY: Peptide
      (B) LOCATION: 5
      (D) OTHER INFORMATION: /label- OMe
   (xi) SEQUENCE DESCRIPTION: SEQ ID NO:1:
   Arg Gly Asp Phe Val
   1               5
(2) INFORMATION FOR SEQ ID NO:2:
   (i) SEQUENCE CHARACTERISTICS:
      (A) LENGTH: 5 amino acids
      (B) TYPE: amino acid
      (D) TOPOLOGY: linear
   (ii) MOLECULE TYPE: peptide
   (v) FRAGMENT TYPE: internal
   (ix) FEATURE:
      (A) NAME/KEY: Peptide
      (B) LOCATION: 1
      (D) OTHER INFORMATION: /label- BOC
   (ix) FEATURE:
      (A) NAME/KEY: Peptide
      (B) LOCATION: 4
      (D) OTHER INFORMATION: /label= D-Phe
         /note- "A prefix "D" in D-Phe signifies that the phenyalanine in position 4 is a D-amino acid."
   (ix) FEATURE:
      (A) NAME/KEY: Peptide
      (B) LOCATION: 5
      (D) OTHER INFORMATION: /label- OH
   (xi) SEQUENCE DESCRIPTION: SEQ ID NO:2:
   Arg Gly Asp Phe Val
   1               5
(2) INFORMATION FOR SEQ ID NO:3:
   (i) SEQUENCE CHARACTERISTICS:
      (A) LENGTH: 5 amino acids
      (B) TYPE: amino acid
      (D) TOPOLOGY: linear
   (ii) MOLECULE TYPE: peptide
   (v) FRAGMENT TYPE: internal
   (ix) FEATURE:
      (A) NAME/KEY: Peptide
      (B) LOCATION: 1
      (D) OTHER INFORMATION: /label- H
   (ix) FEATURE:
      (A) NAME/KEY: Peptide
      (B) LOCATION: 4
      (D) OTHER INFORMATION: /label- D-Phe
         /note- "A prefix "D" in D-Phe signifies that the phenyalanine in position 4 is a D-amino acid."
   (ix) FEATURE:
      (A) NAME/KEY: Peptide
      (B) LOCATION: 5
      (D) OTHER INFORMATION: /label= OH
   (xi) SEQUENCE DESCRIPTION: SEQ ID NO:3:
   Arg Gly Asp Phe Val
   1               5
(2) INFORMATION FOR SEQ ID N0:4:
   (i) SEQUENCE CHARACTERISTICS:
      (A) LENGTH: 5 amino acids
      (B) TYPE: amino acid
      (D) TOPOLOGY: circular
   (ii) MOLECULE TYPE: peptide
   (v) FRAGMENT TYPE: internal
   (ix) FEATURE:
      (A) NAME/KEY: Peptide
      (B) LOCATION: 1
      (D) OTHER INFORMATION: /label- cyclo
   (ix) FEATURE:
      (A) NAME/KEY: Peptide
      (B) LOCATION: 4
      (D) OTHER INFORMATION: /label- D-Phe
         /note- "A prefix "D" in D-Phe signifies that the phenyalanine in position 4 is a D-amino acid."
   (xi) SEQUENCE DESCRIPTION: SEQ ID NO:4:
(2) INFORMATION FOR SEQ ID N0:5:
   (i) SEQUENCE CHARACTERISTICS:
      (A) LENGTH: 5 amino acids
      (B) TYPE: amino acid
      (D) TOPOLOGY: circular
   (ii) MOLECULE TYPE: peptide
   (v) FRAGMENT TYPE: internal
   (ix) FEATURE:
      (A) NAME/KEY: Peptide
      (B) LOCATION: 1
      (D) OTHER INFORMATION: /label= cyclo
   (ix) FEATURE:
      (A) NAME/KEY: Peptide
      (B) LOCATION: 4
      (D) OTHER INFORMATION: /label- D-Phe
         /note= "A prefix "D" in D-Phe signifies that the phenyalanine in position 4 is a D-amino acid."
   (xi) SEQUENCE DESCRIPTION: SEQ ID NO:5:
(2) INFORMATION FOR SEQ ID N0:6:
   (i) SEQUENCE CHARACTERISTICS:
      (A) LENGTH: 6 amino acids
      (B) TYPE: amino acid
      (D) TOPOLOGY: circular
   (ii) MOLECULE TYPE: peptide
   (v) FRAGMENT TYPE: internal
   (ix) FEATURE:
      (A) NAME/KEY: Peptide
      (B) LOCATION: 1
      (D) OTHER INFORMATION: /label- cyclo
   (ix) FEATURE:
      (A) NAME/KEY: Peptide
      (B) LOCATION: 2
      (D) OTHER INFORMATION: /label= D-Arg
         /note- "A prefix "D" in D-Arg signifies that the arginine in position 2 is a D-amino acid."
   (xi) SEQUENCE DESCRIPTION: SEQ ID N0:6:
(2) INFORMATION FOR SEQ ID NO:7:
   (i) SEQUENCE CHARACTERISTICS:
      (A) LENGTH: 5 amino acids
      (B) TYPE: amino acid
      (D) TOPOLOGY: circular
   (ii) MOLECULE TYPE: peptide
   (v) FRAGMENT TYPE: internal
   (ix) FEATURE:
      (A) NAME/KEY: Peptide
      (B) LOCATION: 1
      (D) OTHER INFORMATION: /label- cyclo
   (ix) FEATURE:
      (A) NAME/KEY: Peptide
      (B) LOCATION: 5
      (D) OTHER INFORMATION: /label= D-Val
         /note= "A prefix "D" in D-Val signifies that the valine in position 5 is a D-amino acid."
   (xi) SEQUENCE DESCRIPTION: SEQ ID NO:7:
   Arg Gly Asp Phe Val
   1               5
(2) INFORMATION FOR SEQ ID N0:8:
   (i) SEQUENCE CHARACTERISTICS:
      (A) LENGTH: 15 amino acids
      (B) TYPE: amino acid
      (D) TOPOLOGY: linear
   (ii) MOLECULE TYPE: peptide
   (xi) SEQUENCE DESCRIPTION: SEQ ID N0:8:
(2) INFORMATION FOR SEQ ID NO:9:
   (i) SEQUENCE CHARACTERISTICS:
      (A) LENGTH: 6 amino acids
      (B) TYPE: amino acid
      (D) TOPOLOGY: linear
   (ii) MOLECULE TYPE: peptide
   (v) FRAGMENT TYPE: internal
   (ix) FEATURE:
      (A) NAME/KEY: Peptide
      (B) LOCATION: 4
      (D) OTHER INFORMATION: /label= D-phe
         /note- "A prefix "D" in D-Phe signifies that the phenyalanine in position 4 is a D-amino acid."
   (ix) FEATURE:
      (A) NAME/KEY: Peptide
      (B) LOCATION: 6
      (D) OTHER INFORMATION: /label- MeVal
         /note- "A prefix "Me" in MeVal signifies that the valine in position 6 is a methylated valine."
   (xi) SEQUENCE DESCRIPTION: SEQ ID N0:9:

## Description

### Technical Field

The present invention relates generally to the field of medicine, and relates specifically to methods and compositions for inhibiting αᵥβ₅-mediated angiogenesis of tissues using antagonists of the vitronectin receptor αᵥβ₅.

### Background

Integrins are a class of cellular receptors known to bind extracellular matrix proteins, and therefore mediate cell-cell and cell-extracellular matrix interactions, referred generally to as cell adhesion events. However, although many integrins and their respective ligands are described in the literature, the biological function of many of the integrins remains elusive. The integrin receptors constitute a family of proteins with shared structural characteristics of noncovalent heterodimeric glycoprotein complexes formed of α and β subunits.

The vitronectin receptor, named for its original characteristic of preferential binding to vitronectin, is now known to refer to three different integrins, designated αᵥβ₁, αᵥβ₃ and αᵥβ₅. Horton, Int. J. Exp. Pathol., 71:741-759 (1990). αᵥβ₁ binds fibronectin and vitronectin. αᵥβ₃ binds a large variety of ligands, including fibrin, fibrinogen, laminin, thrombospondin, vitronectin, von Willebrand's factor, osteospontin and bone sialoprotein I. αᵥβ₅ binds vitronectin. The specific cell adhesion roles these three integrins play in the many cellular interactions in tissues are still under investigation. However, it is clear that there are different integrins with different biological functions as well as different integrins and subunits having shared biological specificities.

One important recognition site in a ligand for many integrins is the arginine-glycine-aspartic acid (RGD) tripeptide sequence. RGD is found in all of the ligands identified above for the vitronectin receptor integrins. This RGD recognition site can be mimicked by polypeptides ("peptides") that contain the RGD sequence, and such RGD peptides are known inhibitors of integrin function. It is important to note, however, that depending upon the sequence and structure of the RGD peptide, the specificity of the inhibition can be altered to target specific integrins.

For discussions of the RGD recognition site, see Pierschbacher et al., Nature, 309:30-33 (1984), and Pierschbacher et al., Proc. Natl. Acad. Sci. USA, 81:5985-5988 (1984). Various RGD polypeptides of varying integrin specificity have also been described by Grant et al., Cell, 58:933-943 (1989), Cheresh, et al., Cell, 58:945-953 (1989), Aumailley et al., FEBS Letts., 291:50-54 (1991), and Pfaff et al., J. Biol. Chem., 269:20233-20238 (1994), and in United States Patent Nos. 4,517,686, 4,578,079, 4,589,881, 4,614,517, 4,661,111, 4,792,525, 4,683,291, 4,879,237, 4,988,621, 5,041,380 and 5,061,693.

EP-A-0578083 discloses the use of cyclic RGD-containing polypeptides to inhibit adhesion.

Angiogenesis, also referred to as neovascularization, is a process of tissue vascularization that involves the growth of new developing blood vessels into a tissue. The process is mediated by the infiltration of endothelial cells and smooth muscle cells. The process is believed to proceed in any one of three ways: 1) The vessels can sprout from pre-existing vessels; 2) De novo development of vessels can arise from precursor cells (vasculogenesis); or 3) Existing small vessels can enlarge in diameter. Blood et al., Bioch. Biophys. Acta, 1032:89-118 (1990). Vascular endothelial cells are known to contain at least five RGD-dependent integrins, including the vitronectin receptor (αᵥβ₃ or αᵥβ₅), the collagen Types I and IV receptor (α₁β₁), the laminin receptor (α₂β₁), the fibronectin/laminin/collagen receptor (α₃β₁) and the fibronectin receptor (α₅β₁). Davis et al., J. Cell. Biochem., 51:206-218 (1993). The smooth muscle cell is known to contain at least six RGD-dependent integrins, including α₅β₁, αᵥβ₃ and αᵥβ₅.

Angiogenesis is an important process in neonatal growth, but is also important in wound healing and in the pathogenesis of a large variety of clinically important diseases including tissue inflammation, arthritis, psoriasis, cancer, diabetic retinopathy, macular degeneration and other neovascular eye diseases. These clinical entities associated with angiogenesis are referred to as angiogenic diseases. Folkman et al., Science, 235:442-447 (1987). Angiogenesis is generally absent in adult or mature tissues, although it does occur in wound healing and in the corpus luteum growth cycle. See, for example, Moses et al., Science, 248:1408-1410 (1990).

Inhibition of cell adhesion in vitro using monoclonal antibodies immunospecific for various integrin α or β subunits have implicated the vitronectin receptor αᵥβ₃ in cell adhesion of a variety of cell types including microvascular endothelial cells. Davis et al., J. Cell. BioI., 51:206-218 (1993). In addition, Nicosia et al., Am. J. Pathol., 138:829-833 (1991), described the use of the RGD peptide, GRGDS, to inhibit the in vitro formation of "microvessels" from rat aorta cultured in collagen gel.

However, the inhibition of formation of "microvessels" in vitro in collagen gel cultures is not a model for inhibition of angiogenesis in a tissue because it is not shown that the microvessel structures are the same as capillary sprouts or that the formation of the microvessel in collagen gel culture is the same as neo-vascular growth into an intact tissue, such as arthritic tissue, tumor tissue or disease tissue where inhibition of angiogenesis is desirable.

WO 95/14714 and WO 95/25543 disclose the use of RGD-containing polypeptides for inhibiting αᵥβ₃-mediated angiogenesis.

The role of αᵥβ₃ in angiogenesis was recently confirmed. See, Brooks, et al. Science, 264:569-571 (1994). The integrin was shown to be expressed on blood vessels in human wound granulation tissue but not in normal skin. Monoclonal antibodies against the αᵥβ₃ receptor inhibited angiogenesis induced by the growth factors (cytokines) basic fibroblast growth factor (bFGF) and tumor necrosis factor-α (TNF-α), as well as by melanoma fragments. However, the antagonists only inhibited new and not preexisting vessels. In addition, specific linear and cyclic RGD-containing peptides were also shown to inhibit neovascularization.

It has been proposed that inhibition of angiogenesis would be a useful therapy for restricting tumor growth. Inhibition of angiogenesis has been proposed by (1) inhibition of release of "angiogenic molecules" such as bFGF (basic fibroblast growth factor), (2) neutralization of angiogenic molecules, such as by use of anti-bFGF antibodies, and (3) inhibition of endothelial cell response to angiogenic stimuli. This latter strategy has received attention, and Folkman et al., Cancer Biology, 3:89-96 (1992), have described several endothelial cell response inhibitors, including collagenase inhibitor, basement membrane turnover inhibitors, angiostatic steroids, fungal-derived angiogenesis inhibitors, platelet factor 4, thrombospondin, arthritis drugs such as D-penicillamine and gold thiomalate, vitamin D₃ analogs, alpha-interferon, and the like that might be used to inhibit angiogenesis. For additional proposed inhibitors of angiogenesis, see Blood et al., Bioch. Biophys. Acta., 1032:89-118 (1990), Moses et al., Science, 248:1408-1410 (1990), Ingber et al., Lab. Invest., 59:44-51 (1988), and United States Patent Nos. 5,092,885, 5,112,946, 5,192,744, and 5,202,352.

However, the role of the integrin αᵥβ₅ in angiogenesis has neither been suggested or identified until the present invention nor have any of the inhibitors of angiogenesis described in the foregoing references been targeted at inhibition of αᵥβ₅. Moreover, no references, other than the present invention, have implicated the αᵥβ₅ integrin in neovascularization, particularly that induced by the growth factors, vascular endothelial growth factor (VEGF), transforming growth factor-α (TGF-α) and epidermal growth factor (EGF).

Although the numbers of growth factors involved in the control of angiogenesis are limited, different levels of control of the process exist for conversion of a quiescent state to a neovascular state. See, D'Amore, Investigative Ophthal. Visual Sci., 35:3974-3979 (1994). While some growth factors involved in angiogenesis are regulated at the synthesis level, others are regulated by the state of activation. These cellular events occur as a quiescent vessel undergoes neovascularization following injury or ischemia.

VEGF, in particular, is thought to be a major mediator of angiogenesis in a primary tumor and in ischemic ocular diseases. For review, see Folkman, Nature Medicine, 1:27-31 (1995). VEGF is a 46 kilodalton (kDa) homodimer that is an endothelial cell-specific angiogenic (Ferrara et al., Endocrin. Rev., 13:18-32 (1992)) and vasopermeable factor (Senger et al., Cancer Res., 46:5629-5632 (1986)) that binds to high-affinity membrane-bound receptors with tyrosine kinase activity (Jakeman et al., J. Clin. Invest., 89:244-253 (1992)).

Activation of receptor tyrosine kinases has recently been shown to promote integrin-dependent cell migration on extracellular matrix proteins. In particular, Klemke et al., J. Cell Biol., 127:859-866 (1994) have implicated the EGF receptor (EGFR) tyrosine kinase in promoting cell motility but not adhesion of FG human pancreatic carcinoma cells on vitronectin using the αᵥβ₅ integrin. The authors provide direct evidence that occupation of EGFR with the EGF ligand activates the tyrosine kinase activation of the EGFR that ultimately stimulates a protein kinase C (PKC)-dependent pathway leading to the induction of αᵥβ₅-dependent cell migration of a vitronectin substrate on which the cells are normally unable to migrate. Thus, the Klemke et al. findings provide evidence for correlating the presence of cytokines, specifically EGF, with integrin activity in cell migration. Activation of PKC has been shown to be involved in the regulation of angiogenesis in the chick chorioallantoic membrane model system. See, Tsopanoglou et al., J. Vasc. Res., 30:202-208 (1993). The authors identified specific activators and inhibitors of PKC that respectively stimulated and inhibited angiogenesis in the model system.

However, neither Klemke et al. nor Tsopanoglou et al. discussed above describe the role of cytokines and expression and/or activation of the αᵥβ₅ integrin in promoting angiogenesis in various conditions and disease states and inhibition thereof with αᵥβ₅-specific antagonists.

Recent experimental evidence has shown in a monkey model system of eye disease that retinal ischemia induced by retinal vein occlusion resulted in a rapid rise of VEGF in the aqueous chambers of the eye. This rise coincided with the iris neovascularization that was observed as described by Miller et al., Am. J. Path., 145:574-584 (1994). Additional data in an mouse model system of proliferative retinopathy in which hypoxia is induced, VEGF messenger RNA was shown to increase within 6-12 hours of relative hypoxia that remained elevated until neovascularization developed. As the new blood vessels declined, so did the VEGF expression as described by Pierce et al., Proc. Natl. Acad. Sci.. USA, 92:905-909 (1995).

Thus, the recent data as demonstrated in animal models of ischemia have correlated the induction of VEGF with that of ischemia followed by neovascularization. VEGF, as well as other growth factors, have also been implicated in other conditions and disease states involving neovascularization as reviewed by Folkman, Nature-Medicine, 1:27-31 (1995).

The Folkman et al. reference also summarizes the current clinical approaches used to control undesirable angiogenesis. Patients in clinical trials have received therapeutic treatments with angiogenic inhibitors including platelet factor 4, a fumagillin-derivative, carboxy-amino-triazole, and the like. However, no references or current therapeutic references correlate the expression of αᵥβ₅ with angiogenesis, particularly that induced by VEGF. Thus, prior to the present invention, no one has described nor utilized a therapeutic regimen with αᵥβ₅ antagonists to control angiogenesis in a tissue undergoing angiogenesis correlated with the presence and activation of αᵥβ₅.

Therefore, other than the studies reported here on αᵥβ₃ and the relationship with growth factors to angiogenesis, Applicants are unaware of any other demonstration that angiogenesis could be inhibited in a tissue using inhibitors of αᵥβ₅-mediated cell adhesion. In particular, it has never been previously demonstrated that αᵥβ₅ function is required for angiogenesis in a tissue or that αᵥβ₅ antagonists can inhibit angiogenesis in a tissue, particularly in ocular neovascular diseases.

### Brief Description of the Invention

The present invention demonstrates that in addition to an αᵥβ₃-requiring angiogenesis pathway in tissues, a separate novel αᵥβ₅-dependent pathway also exists. Thus, the invention describes inhibitors of αᵥβ₅ that can inhibit angiogenesis. The invention further describes that αᵥβ₅-mediated activity in promoting angiogenesis is correlated with growth factor (cytokine) activation of growth factor receptor tyrosine kinases and protein kinase C (PKC). The growth factors (cytokines) that function in this manner include vascular endothelial growth factor (VEGF), transforming growth factor-α (TGF-α), epidermal growth factor (EGF), and the like.

The invention is defined in claim 1.

An αᵥβ₅ antagonist for use in the present methods is capable of binding to αᵥβ₅ and competitively inhibiting the ability of αᵥβ₅ to bind to the natural vitronectin ligand. Preferably, the antagonist exhibits specificity for αᵥβ₅ over other integrins. In a particularly preferred embodiment, the αᵥβ₅ antagonist inhibits binding of vitronectin or other RGD-containing ligands to αᵥβ₅ but not substantially inhibit binding of vitronectin to αᵥβ₃ or α_{IIb}β₃.

The αᵥβ₅ antagonist is an RGD-containing polypeptide.

Administration of the αᵥβ₅ antagonists of this invention includes intraocular, administration.

### Brief Description of the Drawings

In the drawings forming a portion of this disclosure:

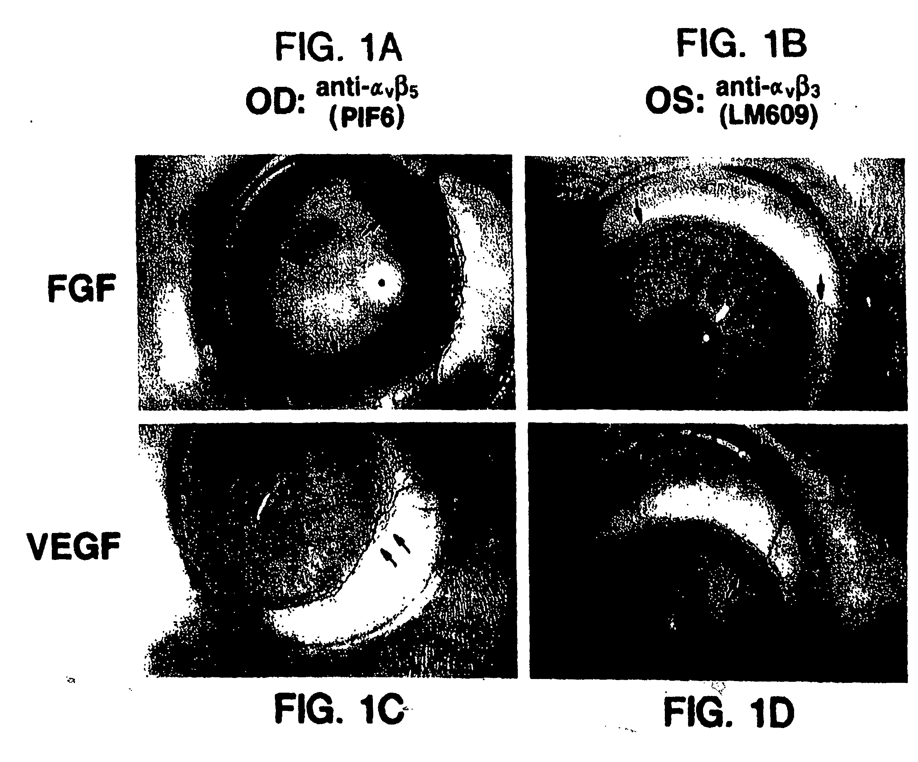

Figures 1A-1D illustrate inhibition of cytokine-induced rabbit corneal angiogenesis by αᵥ integrin antibody antagonists. Induction of angiogenesis by treatment with either bFGF or VEGF and effects of treatment thereof with the αᵥ integrin antibody antagonists, P1F6 (αᵥβ₅) and LM609 (αᵥβ₃), are described in Example 4. OD and OS are respectively the right and left eyes of an experimental rabbit. Large arrows indicate corneal angiogenesis with edema while small arrows point to normal conjunctival limbal vessels. Figures 1A and 1B show induction of angiogenesis with bFGF while Figures 1C and 1D show that with VEGF. Rabbit corneas in Figures 1A and 1C show treatment with P1F6 while Figures 1B and 1D show treatment with LM609.
Figures 2A and 2B are histograms showing the mean neovascular area in mm² +/- the standard error (n = 8 for each of two series) after induction respectively with either bFGF or VEGF followed by mAb treatment with either P1F6 or LM609. The results are discussed in Example 4.

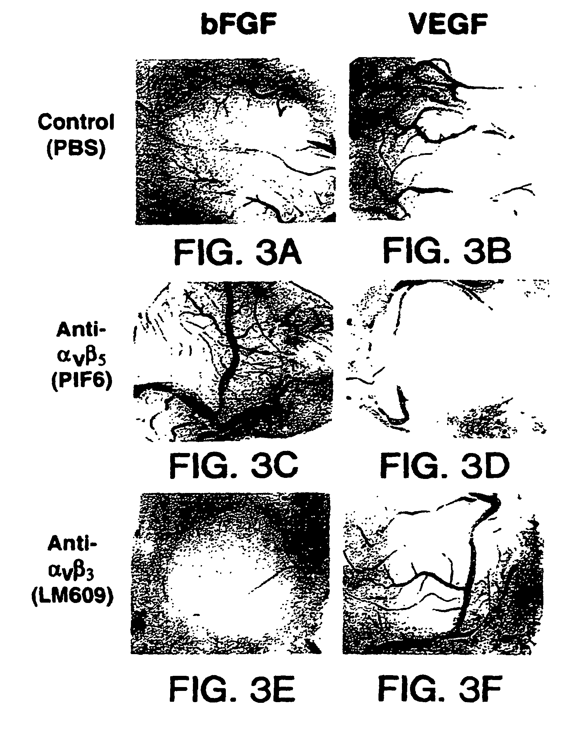

Figures 3A-3F photographically illustrate the effects of anti-integrin antibody treatment on the chick CAM preparation. The results are described in Example 6A. Angiogenesis is either induced with bFGF or VEGF followed by intravenous administration of phosphate buffered saline (PBS) as a control or with P1F6 or LM609 monoclonal antibodies described in the legend for Figure 1. CAMs treated with bFGF are shown in Figures 3A, 3C and 3E while CAMs treated with VEGF are shown in Figures 3B, 3D and 3F. Control CAMs receiving intravenous injections of PBS are shown in Figures 3A and 3B. The P1F6 antibody was used to treat CAMs shown in Figures 3C and 3D while the LM609 antibody was used to treat CAMs in Figures 3E and 3F.
Figures 4A and 4B provide in histogram format the quantitation of results shown in Figures 3A-3F. The angiogenesis index is plotted on the Y-axis against control or antibody treatment. Figures 4A and 4B respectively show bFGF- and VEGF-induced angiogenesis. The results are discussed in Example 4.

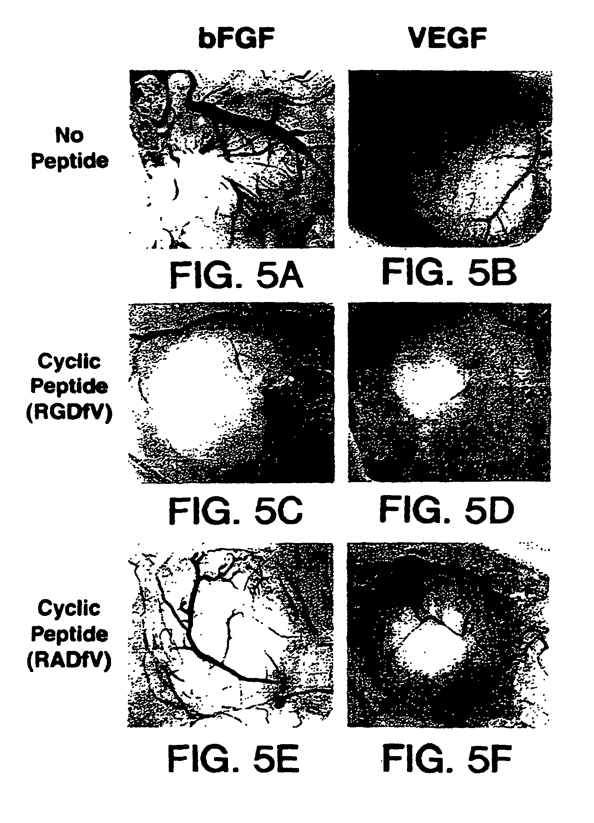

Figures 5A-5F photographically illustrate the effects of synthetic peptide treatment on the chick CAM preparation as described in Example 6. Angiogenesis is either induced with bFGF or VEGF followed by intravenous administration of phosphate buffered saline (PBS) as a control or with the synthetic cyclic peptides RGDfV (SEQ ID NO 4) or RADfV (SEQ ID NO 5). CAMs treated with bFGF are shown in Figures 5A, 5C and 5E while CAMs treated with VEGF are shown in Figures 5B, 5D and 5F. Control CAMs receiving intravenous injections of PBS are shown in Figures 5A and 5B. The RDGfV peptide was used to treat CAMs shown in Figures 5C and 5D while the RADfV peptide was used to treat CAMs in Figures 5E and 5F.
Figures 6A and 6B provide, in histogram format, the quantitation of results shown in Figures 5A-5F. The angiogenesis index is plotted on the Y-axis against control or antibody treatment. Figures 6A and 6B respectively show bFGF- and VEGF-induced angiogenesis. The results are discussed in Example 6.
Figures 7A-7E show the effects of anti-integrin monoclonal antibodies and calphostin C on CAM angiogenesis induced by the separate cytokines, bFGF, TNF-α, VEGF and TGF-α. PMA was also evaluated. The assays and results are described in Example 6. The results are plotted in histogram format where angiogenesis index is graphed on the Y-axis and the various control or inhibitors are shown on the X-axis. Figures 7A-7E respectively show angiogenesis induced with bFGF, TNF-α, VEGF, TGF-α and PMA.
Figure 8 is a histogram showing the effects of antibody treatment on CS1 melanoma tumor growth in the chick embryo CAM assayed performed as described in Examples 5C and 6D. The weight of the tumors in milligrams (mg) is plotted on the Y-axis against the various treatments indicated on X-axis. CSAT is a control antibody specific for the integrin β1 subunit. LM609 and P1F6 are previously described.
Figure 9 is a histogram of the effects of control versus an αᵥβ₅ peptide antagonist, labeled peptide 189 (SEQ ID NO 9) on melanoma tumor growth as measured by tumor volume in mm³ plotted on the Y-axis. The assay and results are described in Example 8.

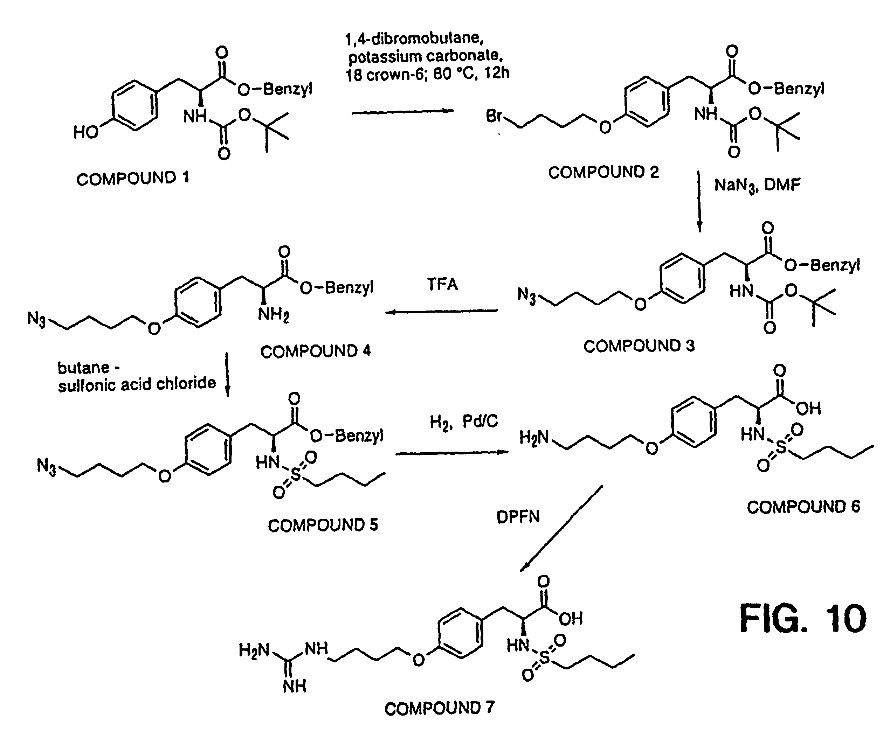

Figure 10 illustrates the synthesis of Compound 7 as described in Example 10A-G.

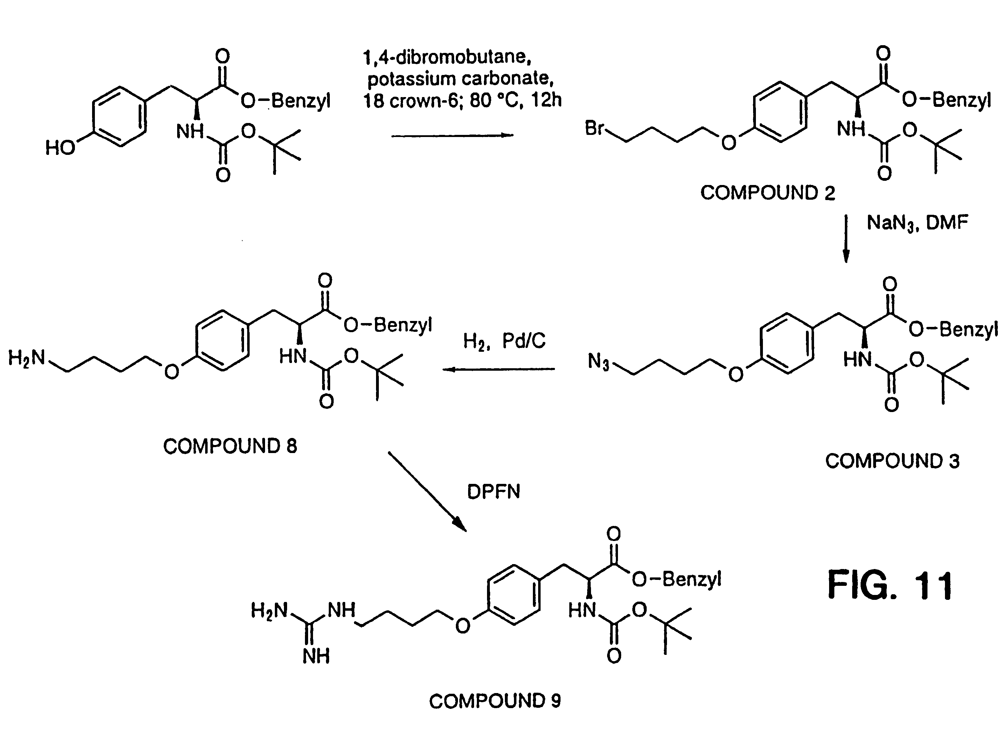

Figure 11 illustrates the synthesis of Compound 9 as described in Example 10A-C; H-I.

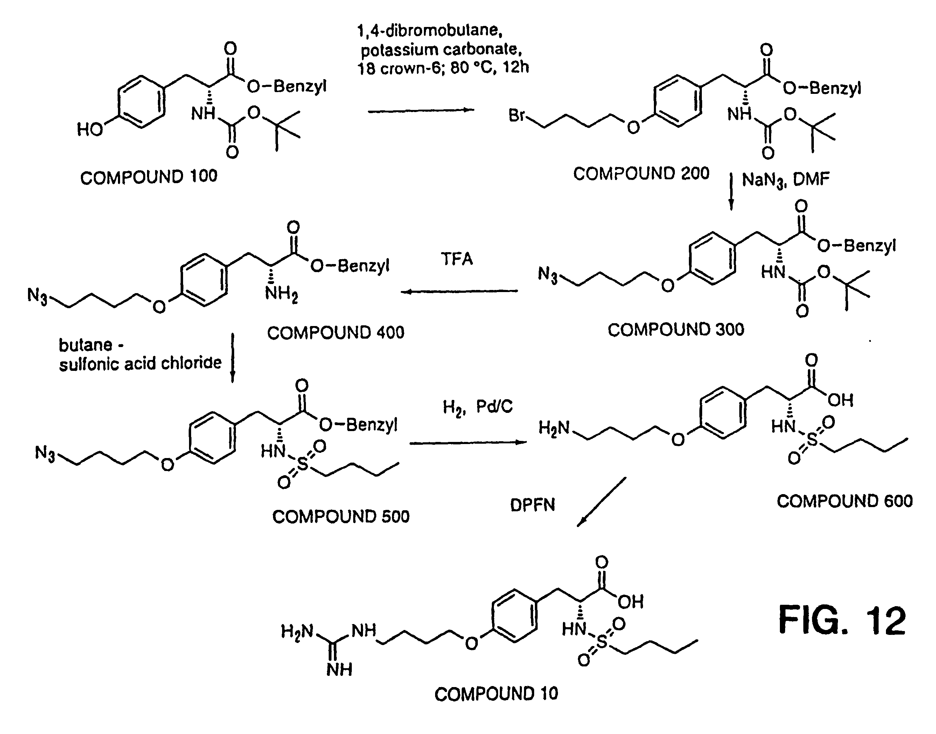

Figure 12 illustrates the synthesis of Compound 10 as described in Example 10J.

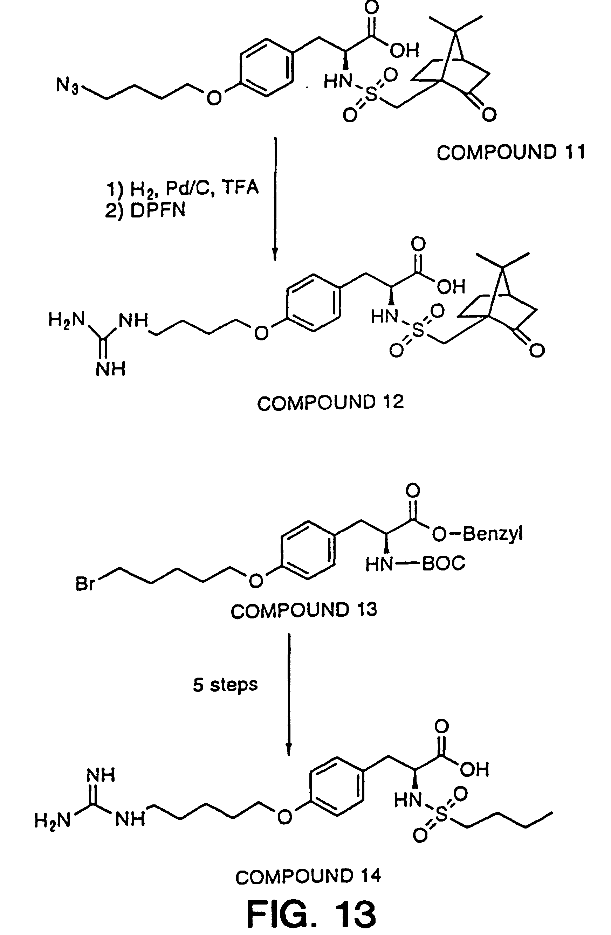

Figure 13 illustrates the synthesis of Compound 12 and Compound 14 as respectively described in Example 10K-L and 10M-N.

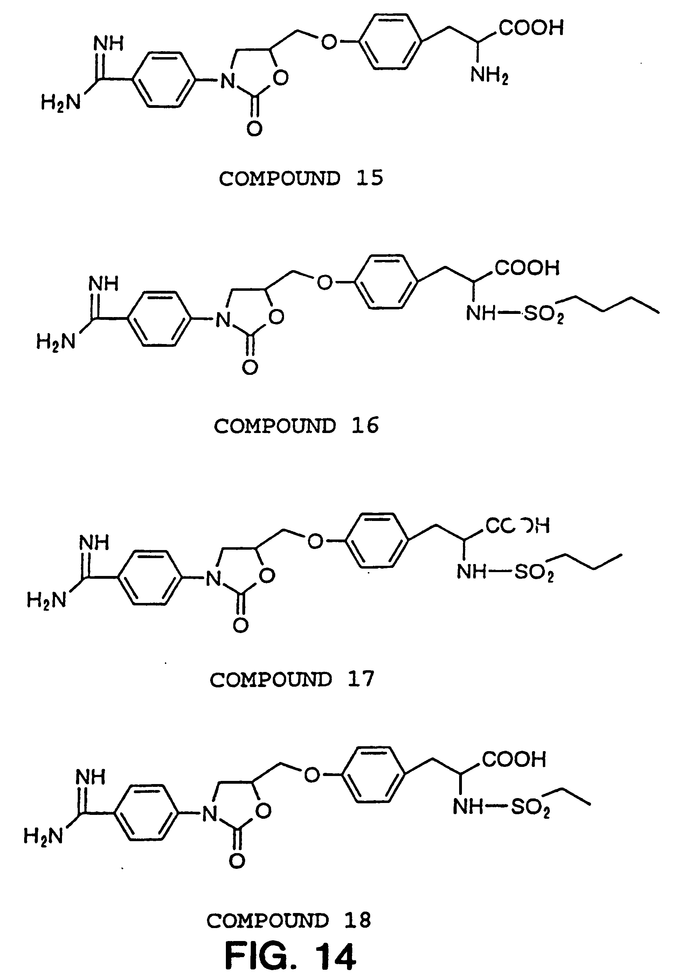

Figure 14 shows the chemical structures of Compound 15, Compound 16, Compound 17 and Compound 18. The detailed synthesis of said compounds are described in Example 100-R.

### Detailed Description of the Invention

### A. Definitions

Amino Acid Residue: An amino acid formed upon chemical digestion (hydrolysis) of a polypeptide at its peptide linkages. The amino acid residues described herein are preferably in the "L" isomeric form. However, residues in the "D" isomeric form can be substituted for any L-amino acid residue, as long as the desired functional property is retained by the polypeptide. NH₂ refers to the free amino group present at the amino terminus of a polypeptide. COOH refers to the free carboxy group present at the carboxy terminus of a polypeptide. In keeping with standard polypeptide nomenclature (described in J. Biol. Chem., 243:3552-59 (1969) and adopted at 37 CFR §1.822(b)(2)), abbreviations for amino acid residues are shown in the following Table of Correspondence:

| TABLE OF CORRESPONDENCE | | |
|---|---|---|
| SYMBOL | | AMINO ACID |
| 1-Letter | 3-Letter | |
| Y | Tyr | tyrosine |
| G | Gly | glycine |
| F | Phe | phenylalanine |
| M | Met | methionine |
| A | Ala | alanine |
| S | Ser | serine |
| I | Ile | isoleucine |
| L | Leu | leucine |
| T | Thr | threonine |
| V | Val | valine |
| P | Pro | proline |
| K | Lys | lysine |
| H | His | histidine |
| Q | Gln | glutamine |
| E | Glu | glutamic acid |
| Z | Glx | Glu and/or Gln |
| W | Trp | tryptophan |
| R | Arg | arginine |
| D | Asp | aspartic acid |
| N | Asn | asparagine |
| B | Asx | Asn and/or Asp |
| C | Cys | cysteine |
| X | Xaa | Unknown or other |
| In addition the following have the meanings below: BOC tert-butyloxycarbonyl DCCI dicylcohexylcarbodiimide DMF dimethylformamide OMe methoxy HOBt 1-hydroxybezotriazole | | |

It should be noted that all amino acid residue sequences are represented herein by formulae whose left and right orientation is in the conventional direction of amino-terminus to carboxy-terminus. Furthermore, it should be noted that a dash at the beginning or end of an amino acid residue sequence indicates a peptide bond to a further sequence of one or more amino acid residues.

Polypeptide: A linear series of amino acid residues connected to one another by peptide bonds between the alpha-amino group and carboxy group of contiguous amino acid residues.

Peptide: A linear series of no more than about 50 amino acid residues connected one to the other as in a polypeptide.

Cyclic peptide: A circular peptide derived from a corresponding linear peptide and-refers to a peptide in which no free N- or C-termini exist of which the corresponding linear peptide's N-termini forms an amide bond to the C-terminal carboxylate of the said corresponding linear peptide.

Protein: A linear series of greater than 50 amino acid residues connected one to the other as in a polypeptide.

Synthetic peptide: A chemically produced chain of amino acid residues linked together by peptide bonds that is free of naturally occurring proteins and fragments thereof.

### B. General Considerations

The present invention relates generally to the discovery that angiogenesis is mediated by the specific vitronectin receptor αᵥβ₅, and that inhibition of αᵥβ₅ function inhibits angiogenesis.
This discovery is important because of the role that angiogenesis plays in a variety of disease processes. By inhibiting angiogenesis, one can intervene in the disease, ameliorate the symptoms, and in some cases cure the disease.

Where the growth of new blood vessels is the cause of, or contributes to, the pathology associated with a disease, inhibition of angiogenesis will reduce the deleterious effects of the disease. Examples include rheumatoid arthritis, diabetic retinopathy, inflammatory diseases, restenosis, and the like. Where the growth of new blood vessels is required to support growth of a deleterious tissue, inhibition of angiogenesis will reduce the blood supply to the tissue and thereby contribute to reduction in tissue mass based on blood supply requirements. Examples include growth of new blood vessels in response to ischemia, resulting in growth factor-induced angiogenesis, growth of tumors where neovascularization is a continual requirement in order that the tumor grow beyond a few millimeters in thickness, and for the establishment of solid tumor metastases.

The methods of the present invention are effective in part because the therapy is highly selective for angiogenesis and not other biological processes. As shown in the Examples, only new vessel growth contains substantial αᵥβ₅, and therefore the therapeutic methods do not adversely effect mature vessels.

The discovery that inhibition of αᵥβ₅ alone will effectively inhibit angiogenesis allows for the development of therapeutic compositions with potentially high specificity, and therefore relatively low toxicity. Although the invention discloses the use of peptide-based reagents which have the ability to inhibit one or more integrins, one can design other reagents which more selectively inhibit αᵥβ₅. Therefore, certain peptide-based reagents do not have the side effect of inhibiting other biological processes other that those mediated by αᵥβ₅.

For example, RGD-containing peptides can be designed to be selective for inhibition of αᵥβ₅, as described further herein.

Prior to the discoveries of the present invention, it was not known that angiogenesis, and any of the processes dependent on angiogenesis, could be inhibited in vivo by the use of reagents that antagonize the biological function of αᵥβ₅.

### C. Methods For Inhibition of Angiogenesis

The invention provides for a method of inhibiting angiogenesis in a tissue, and thereby inhibiting events in the tissue which depend upon angiogenesis. Generally, the method comprises administering to the tissue a composition comprising an angiogenesis-inhibiting amount of an αᵥβ₅ antagonist.

The target tissue used in practicing the methods of this invention is defined as αᵥβ₅-containing corneal tissue that is characterized by the detectable presence of αᵥβ₅ integrin receptor. In other words, an αᵥβ₅-containing tissue is defined by the presence of the αᵥβ₅ receptor complex in the cell membranes. Such tissues include epithelially and mesenchymally derived cells. The presence of the receptor can be determined by a number of means including immunoreactivity of the receptor with an anti-αᵥβ₅ integrin receptor antibody, wherein the immunoreaction is detected in tissues by microscopy, by immunoprecipitation, by competition in ligand binding assays and the like techniques. Preferred antibodies for use in detecting the presence of αᵥβ₅ in a tissue are described below and in Example 1. For example, the distribution of αᵥβ₅ in kidney, skin and ocular tissues by immunofluorescence microscopy is described in Example 2.

In the context of the methods of this invention, an αᵥβ₅-containing tissue is also characterized as one that has an indicia of angiogenesis. As described earlier, angiogenesis includes a variety of processes involving neovascularization of a tissue including "sprouting", vasculogenesis, or vessel enlargement, all of which angiogenesis processes are mediated by and dependent upon the expression of αᵥβ₅. With the exception of traumatic wound healing, corpus luteum formation and embryogenesis, it is believed that the majority of angiogenesis processes are associated with disease processes and therefore the use of the present therapeutic methods are selective for the disease and do not have deleterious side effects.

There are a variety of diseases in which angiogenesis is believed to be important, referred to as angiogenic diseases, including but not limited to, inflammatory disorders such as immune and non-immune inflammation, chronic articular rheumatism and psoriasis, disorders associated with inappropriate or inopportune invasion of vessels such as restenosis, capillary proliferation in atherosclerotic plaques and osteoporosis, and cancer associated disorders, such as solid tumors, solid tumor metastases, angiofibromas, retrolental fibroplasia, hemangiomas, Kaposi sarcoma and the like cancers which require neovascularization to support tumor growth.

Eye diseases characterized by neovascularization present a particularly preferred target for therapy. Ocular neovascularization is the most common pathological change observed in the vast majority of eye diseases that result in catastrophic loss of vision. The growth of new blood vessels from the preexisting choroidal, retinal or paralimbal vessels can lead to edema, hemorrhage or fibrovascular membrane formation resulting in disruption of the normal anatomic relationships of the eye and concomitant loss of normal visual function.

The present invention specifically concerns certain corneal neovascular disorders, namely corneal transplantation, herpetic keratitis, luetic keratitis, pterygium and neovascular pannus associated with contact lens use.

Thus, methods which inhibit angiogenesis in a diseased tissue ameliorate symptoms of the disease and, depending upon the disease, can contribute to cure of the disease. The extent of angiogenesis in a tissue, and therefore the extent of inhibition achieved by the present methods, can be evaluated by a variety of methods, such as are described in the Examples for detecting αᵥβ₅-immunopositive nascent and immature vessel structures by immunohistochemistry.

In particular, the methods and αᵥβ₅ antagonist compositions of this invention are therapeutically useful for inhibiting angiogenesis that has been induced by growth factors, also referred to as cytokines. Under physiological conditions, angiogenesis is highly regulated and as previously published by Brooks et al., Science, 264:569-5761 (1994), has been shown to be activated by specific angiogenic molecules such as basic fibroblast growth factor (bFGF). Negative regulators of angiogenesis have also been described. Angiogenesis is thus regulated by an intricate balance between local stimulators and inhibitors. See, D'Amore, Investigative Ophthal. Visual Sci., 35:3974-3979 (1994).

When the physiologic balance of angiogenic stimulators and inhibitors that tightly control the normally quiescent capillary vascular is disturbed, as occurs is certain disease states, capillary endothelial cells are induced to proliferate, migrate and ultimately differentiate to form new blood vessels.

Angiogenesis is characterized as an event cascade having a set of early events followed by a set of late events as reviewed by Leibovich, "Role of Cytokines in the Process of Tumor Angiogenesis", in "Human Cytokines: Their Role in Disease and Therapy", eds. Aggarwal and Puri, Chapter 35, Blackwell Science, Inc. (1995). The early events are preceded by the delivery of angiogenic growth factors and cytokines delivered from an extravascular source. The early events then proceed in the target microvasculature with the disruption of intercellular junctions, induction of expression of endothelial cell activation antigens and a proteolytic phenotype, and initiation of endothelial cell migration in a directional manner. The late events are characterized with autocrine and paracrine expression of growth factor and cytokine genes within the cells, endothelial cells, pericytes and smooth muscle cells, of the developing capillary bud. These cells in turn modulate the interactions of the cells with the extracellular matrix resulting in the formation of new functional capillary loops from existing mature vessels.

As discussed herein and in the Background, reports in the literature describe an association between the appearance of growth factors, including those associated with an increase of αᵥβ₅ expression, namely VEGF, TGF-α and EGF, with the expansion of a tumor mass and in the onset of angiogenesis in proliferative neovascular eye diseases, both in humans and experimental animals.

Thus, VEGF, EGF, TGF-α, among many others, are considered growth factors which are characterized by their properties of stimulating cellular growth. Growth factors are proteins that are secreted by one cell that act on the secreting cell or another cell. Their ability to act is dependent on the presence of growth factor receptors that are usually transmembrane proteins. Growth factors such as VEGF are also referred to generally as cytokines that are defined as polypeptide hormones, secreted by a cell, that affect growth and metabolism either of the same (autocrine) or of another (paracrine) cell. The term cytokine is not limited to molecules produced by cells of the immune system and the biological response modifiers of the same system. Thus, the term cytokine is a broad category of which one subcategory based on the type of biological response is stimulatory growth factors or enhancers such as VEGF, bFGF, EGF, TGF-α, and the like. For review see, Aggarwal et al., "Common and Uncommon Features of Cytokines and Cytokine Receptors: An Overview", in "Human Cytokines: Their Role in Disease and Therapy", eds. Aggarwal and Puri, Chapter 1, Blackwell Science, Inc. (1995).

In the present invention, αᵥβ₅-specific antagonists, and not growth factor antagonists such as antibodies against VEGF, are contemplated for use in inhibiting angiogenesis in a tissue. In preferred embodiments, the αᵥβ₅ antagonists described herein are useful for inhibiting growth factor-induced angiogenesis in which the expression of the αᵥβ₅ integrin receptor is induced. Preferred growth factors in this context include VEGF, EGF, TGF-α and the like.

As discussed in the Background, the growth factors EGF and VEGF are both known to bind to their cellular receptors that act as tyrosine kinases. Activation of the EGF receptor has further been shown to be correlated with activation of protein kinase C that results in activation of αᵥβ₅ to allow for migration of specific cells on a vitronectin substrate. Thus, the mechanism of action between exposure to cytokines or growth factors and the coordinate response in integrin expression or activation is a complex biological process. As shown in the present invention (see Example 6A), treatment of tissues in either the rabbit eye model or the chick chorioallantoic model with the cytokine VEGF results in the αᵥβ₅-potentiated angiogenesis that is dependent on activation of protein kinase C.

An exemplary model system for assessing the effects of an αᵥβ₅ antagonist of this invention is the murine model of retinal neovascularization as described in Example 9.

A chimeric mouse:human model in which skin of a mouse having severe combined immunodeficiency (SCID) is replaced with human neonatal foreskin is described by Yan et al., J. Clin. Invest., 91:986-996 (1993). The latter model presents an additional in vivo model to investigate angiogenesis and inhibition thereof with the methods of this invention. Exemplary results with a rabbit tumor model and an αᵥβ₅ antagonist of this invention are presented in Examples 5C and 6D while results for inbition of angiogenesis in the SCID mouse model is described in Example 8.

The patient treated in the present invention in its many embodiments is desirably a human patient, although it is to be understood that the principles of the invention indicate that the invention is effective with respect to all mammals, which are intended to be included in the term "patient". In this context, a mammal is understood to include any mammalian species in which treatment of diseases, particularly agricultural and domestic mammalian species, is sought with respect to the methods of this invention.

The present method for inhibiting angiogenesis in a tissue, and therefore for also practicing the methods for treatment of angiogenesis-related diseases, comprises contacting a tissue in which angiogenesis is occurring, or is at risk for occurring, with a composition comprising a therapeutically effective amount of an αᵥβ₅ antagonist capable of inhibiting αᵥβ₅ binding to its natural ligand. Thus, the method comprises administering to a patient a therapeutically effective amount of a physiologically tolerable composition containing an αᵥβ₅ antagonist of the invention.

The dosage ranges for the administration of the αᵥβ₅ antagonist depend upon the form of the antagonist, and its potency, as described further herein, and are amounts large enough to produce the desired effect in which angiogenesis and the disease symptoms mediated by angiogenesis are ameliorated. The dosage should not be so large as to cause adverse side effects, such as hyperviscosity syndromes, pulmonary edema, congestive heart failure, and the like. Generally, the dosage will vary with the age, condition, sex and extent of the disease in the patient and can be determined by one of skill in the art. The dosage can also be adjusted by the individual physician in the event of any complication.

An αᵥβ₅ antagonist is a molecule that blocks or inhibits the physiologic or pharmacologic activity of αᵥβ₅ by inhibiting the binding activity of the receptor to its ligand, namely vitronectin. The αᵥβ₅ antagonists of the invention are RGD-containing polypeptides.

A therapeutically effective amount is an amount of αᵥβ₅ antagonist sufficient to produce a measurable inhibition of angiogenesis in the tissue being treated, i.e., an angiogenesis-inhibiting amount. Inhibition of angiogenesis can be measured in situ by immunohistochemistry, as described herein, or by other methods known to one skilled in the art.

Insofar as an αᵥβ₅ antagonist can take the form of an RGD-containing peptide, it is to be appreciated that the potency, and therefore an expression of a "therapeutically effective" amount can vary. However, as shown by the present assay methods, one skilled in the art can readily assess the potency of a candidate αᵥβ₅ antagonist of this invention.

Potency of an αᵥβ₅ antagonist can be measured by a variety of means including inhibition of angiogenesis in the CAM assay, in the in vivo rabbit eye assay, and by measuring inhibition of binding of natural ligand to αᵥβ₅, all as described herein, and the like assays.

A preferred αᵥβ₅ antagonist has the ability to substantially inhibit binding of a natural ligand such as vitronectin to αᵥβ₅ in solution at antagonist concentrations of less than 0.5 micromolar (µM), preferably less than 0.1 µM, and more preferably less than 0.05 µM. By "substantially" is meant that at least a 50 percent reduction in binding of vitronectin is observed by inhibition in the presence of the αᵥβ₅ antagonist, and at 50% inhibition is referred to herein as an IC₅₀ value.

A more preferred αᵥβ₅ antagonist exhibits selectivity for αᵥβ₅ over other integrins. Thus, a preferred αᵥβ₅ antagonist substantially inhibits vitronectin binding to αᵥβ₅ but does not substantially inhibit binding of vitronectin to another integrin, such as αᵥβ₁, αᵥβ₃ or α_{IIb}β₃. Particularly preferred is an αᵥβ₅ antagonist that exhibits a 10-fold to 100-fold lower IC₅₀ activity at inhibiting vitronectin binding to αᵥβ₅ compared to the IC₅₀ activity at inhibiting vitronectin binding to another integrin. Exemplary assays for measuring IC₅₀ activity at inhibiting vitronectin binding to an integrin are described in the Examples.

A therapeutically effective amount of an αᵥβ₅ antagonist is typically an amount of polypeptide such that when administered in a physiologically tolerable composition is sufficient to achieve a plasma concentration of from 0.1 microgram (µg) per milliliter (ml) to 200 µg/ml, preferably from 1 µg/ml to 150 µg/ml. Based on a polypeptide having a mass of about 500 grams per mole, the preferred plasma concentration in molarity is from 2 micromolar (µM) to 5 millimolar (mM) and preferably 100 µM to 1 mM polypeptide antagonist. Stated differently, the dosage per body weight can vary from about 0.1 mg/kg to about 300 mg/kg, and preferably from 0.2 mg/kg to 200 mg/kg, in one or more dose administrations daily, for one or several days.

The polypeptides of this invention can be administered parenterally by injection or by gradual infusion over time. Although the tissue to be treated can typically be accessed in the body by systemic administration and therefore most often treated by intravenous administration of therapeutic compositions, other tissues and delivery means are contemplated where there is a likelihood that the tissue targeted contains the target molecule. Thus, polypeptides of this invention can be administered intraocularly, intravenously, intraperitoneally, intramuscularly, subcutaneously, intracavity, transdermally, and can also be delivered by peristaltic means.

The therapeutic compositions containing an αᵥβ₅ antagonist of this invention are conventionally administered intravenously, as by injection of a unit dose, for example. The term "unit dose" when used in reference to a therapeutic composition of the present invention refers to physically discrete units suitable as unitary dosage for the subject, each unit containing a predetermined quantity of active material calculated to produce the desired therapeutic effect in association with the required diluent, i.e., carrier, or vehicle.

In one preferred embodiment as shown in the Examples, the αᵥβ₅ antagonist is administered in a single dosage intravenously.

The compositions are administered in a manner compatible with the dosage formulation and in a therapeutically effective amount. The quantity to be administered and timing of administration depends on the subject to be treated, capacity of the subject's system to utilize the active ingredient, and degree of therapeutic effect desired. Precise amounts of active ingredient required to be administered depend on the judgement of the practitioner and are peculiar to each individual. However, suitable dosage ranges for systemic application are disclosed herein and depend on the route of administration. Suitable regimens for administration are also variable but are typified by an initial administration followed by repeated doses at one or more hour intervals by a subsequent injection or other administration. Alternatively, continuous intravenous infusion sufficient to maintain concentrations in the blood in the ranges specified for in vivo therapies are contemplated.

### D. Therapeutic Compositions

The present invention contemplates therapeutic compositions useful for practicing the therapeutic methods described herein. Therapeutic compositions of the present invention contain a physiologically tolerable carrier together with an αᵥβ₅ antagonist as described herein, dissolved or dispersed therein as an active ingredient. In a preferred embodiment, the therapeutic αᵥβ₅ antagonist composition is not immunogenic when administered to a mammal or human patient for therapeutic purposes.

As used herein, the terms "pharmaceutically acceptable", "physiologically tolerable" and grammatical variations thereof, as they refer to compositions, carriers, diluents and reagents, are used interchangeably and represent that the materials are capable of administration to or upon a mammal without the production of undesirable physiological effects such as nausea, dizziness, gastric upset and the like.

The preparation of a pharmacological composition that contains active ingredients dissolved or dispersed therein is well understood in the art and need not be limited based on formulation. Typically such compositions are prepared as injectables either as liquid solutions or suspensions, however, solid forms suitable for solution, or suspensions, in liquid prior to use can also be prepared. The preparation can also be emulsified.

The active ingredient can be mixed with excipients which are pharmaceutically acceptable and compatible with the active ingredient and in amounts suitable for use in the therapeutic methods described herein. Suitable excipients are, for example, water, saline, dextrose, glycerol, ethanol or the like and combinations thereof. In addition, if desired, the composition can contain minor amounts of auxiliary substances such as wetting or emulsifying agents, pH buffering agents and the like which enhance the effectiveness of the active ingredient.

The therapeutic composition of the present invention can include pharmaceutically acceptable salts of the components therein. Pharmaceutically acceptable salts include the acid addition salts (formed with the free amino groups of the polypeptide) that are formed with inorganic acids such as, for example, hydrochloric or phosphoric acids, or such organic acids as acetic, tartaric, mandelic and the like. Salts formed with the free carboxyl groups can also be derived from inorganic bases such as, for example, sodium, potassium, ammonium, calcium or ferric hydroxides, and such organic bases as isopropylamine, trimethylamine, 2-ethylamino ethanol, histidine, procaine and the like.

Particularly preferred is the HCl salt when used in the preparation of cyclic polypeptide αᵥβ₅ antagonists.

Physiologically tolerable carriers are well known in the art. Exemplary of liquid carriers are sterile aqueous solutions that contain no materials in addition to the active ingredients and water, or contain a buffer such as sodium phosphate at physiological pH value, physiological saline or both, such as phosphate-buffered saline. Still further, aqueous carriers can contain more than one buffer salt, as well as salts such as sodium and potassium chlorides, dextrose, polyethylene glycol and other solutes.

Liquid compositions can also contain liquid phases in addition to and to the exclusion of water. Exemplary of such additional liquid phases are glycerin, vegetable oils such as cottonseed oil, and water-oil emulsions.

A therapeutic composition contains an angiogenesis-inhibiting amount of an αᵥβ₅ antagonist of the present invention, typically formulated to contain an amount of at least 0.1 weight percent of antagonist per weight of total therapeutic composition. A weight percent is a ratio by weight of inhibitor to total composition. Thus, for example, 0.1 weight percent is 0.1 grams of inhibitor per 100 grams of total composition.

### E. Antagonists of Integrin αᵥβ₅

αᵥβ₅ antagonists are used in the present methods for inhibiting angiogenesis in tissues, and can take a variety of forms that include compounds which interact with αᵥβ₅ in a manner such that functional interactions with the natural αᵥβ₅ ligands are interfered.

### 1. Polypeptides

The invention contemplates αᵥβ₅ antagonists in the form of RGD-containing polypeptides. A polypeptide (peptide) αᵥβ₅ antagonist can have the sequence characteristics of either the natural ligand of αᵥβ₅ or αᵥβ₅ itself at the region involved in αᵥβ₅-ligand interaction and exhibits αᵥβ₅ antagonist activity as described herein. A preferred αᵥβ₅ antagonist peptide corresponds in sequence to the natural ligand in the RGD-containing region.

Preferred RGD-containing polypeptides have a sequence corresponding to the amino acid residue sequence of the RGD-containing region of a natural ligand of αᵥβ₅ such as vitronectin, for which the sequence is well known.

A particularly preferred αᵥβ₅ antagonist peptide preferentially inhibits αᵥβ₅ binding to its natural ligand(s) when compared to other integrins, as described earlier. These αᵥβ₅-specific peptides are particularly preferred at least because the specificity for αᵥβ₅ reduces the incidence of undesirable side effects such as inhibition of other integrins. The identification of preferred αᵥβ₅ antagonist peptides having selectivity for αᵥβ₅ can readily be identified in a typical inhibition of binding assay, such as the ELISA assay described in the Examples.

In one embodiment, a polypeptide of the present invention comprises no more than about 100 amino acid residues, preferably no more than about 60 residues, more preferably no more than about 30 residues. Peptides can be linear or cyclic, although particularly preferred peptides are cyclic. Preferred peptides are described in the Examples.

It should be understood that a subject polypeptide need not be identical to the amino acid residue sequence of a αᵥβ₅ natural ligand, so long as it includes a sequence necessary for antagonizing the binding of an αᵥβ₅ ligand to αᵥβ₅ and is able to function as an αᵥβ₅ antagonist in an assay such as those described herein.

A subject polypeptide includes any analog, fragment or chemical derivative of a polypeptide whose amino acid residue sequence is shown herein so long as the polypeptide is an αᵥβ₅ antagonist. Therefore, a present polypeptide can be subject to various changes, substitutions, insertions, and deletions where such changes provide for certain advantages in its use. In this regard, an αᵥβ₅ antagonist polypeptide of this invention corresponds to, rather than is identical to, the sequence of a recited peptide where one or more changes are made and it retains the ability to function as an αᵥβ₅ antagonist in one or more of the assays as defined herein.

Thus, a polypeptide can be in any of a variety of forms of peptide derivatives, that includes amides, conjugates with proteins, cyclized peptides, polymerized peptides, analogs, fragments, chemically modified peptides, and the like derivatives.

The term "analog" includes any polypeptide having an amino acid residue sequence substantially identical to a sequence specifically shown herein in which one or more residues have been conservatively substituted with a functionally similar residue and which displays the αᵥβ₅ antagonist activity as described herein. Examples of conservative substitutions include the substitution of one non-polar (hydrophobic) residue such as isoleucine, valine, leucine or methionine for another, the substitution of one polar (hydrophilic) residue for another such as between arginine and lysine, between glutamine and asparagine, between glycine and serine, the substitution of one basic residue such as lysine, arginine or histidine for another, or the substitution of one acidic residue, such as aspartic acid or glutamic acid for another.

The phrase "conservative substitution" also includes the use of a chemically derivatized residue in place of a non-derivatized residue provided that such polypeptide displays the requisite inhibition activity.

"Chemical derivative" refers to a subject polypeptide having one or more residues chemically derivatized by reaction of a functional side group. Such derivatized molecules include for example, those molecules in which free amino groups have been derivatized to form amine hydrochlorides, p-toluene sulfonyl groups, carbobenzoxy groups, t-butyloxycarbonyl groups, chloroacetyl groups or formyl groups. Free carboxyl groups may be derivatized to form salts, methyl and ethyl esters or other types of esters or hydrazides. Free hydroxyl groups may be derivatized to form O-acyl or 0-alkyl derivatives. The imidazole nitrogen of histidine may be derivatized to form N-imbenzylhistidine. Also included as chemical derivatives are those peptides which contain one or more naturally occurring amino acid derivatives of the twenty standard amino acids. For example: 4-hydroxyproline may be substituted for proline; 5-hydroxylysine may be substituted for lysine; 3-methylhistidine may be substituted for histidine; homoserine may be substituted for serine; and ornithine may be substituted for lysine. Polypeptides of the present invention also include any polypeptide having one or more additions and/or deletions or residues relative to the sequence of a polypeptide whose sequence is shown herein, so long as the requisite activity is maintained.

The term "fragment" refers to any subject polypeptide having an amino acid residue sequence shorter than that of a polypeptide whose amino acid residue sequence is shown herein.

When a polypeptide of the present invention has a sequence that is not identical to the sequence of an αᵥβ₅ natural ligand, it is typically because one or more conservative or non-conservative substitutions have been made, usually no more than about 30 number percent, and preferably no more than 10 number percent of the amino acid residues are substituted. Additional residues may also be added at either terminus of a polypeptide for the purpose of providing a "linker" by which the polypeptides of this invention can be conveniently affixed to a label or solid matrix, or carrier.

Labels, solid matrices and carriers that can be used with the polypeptides of this invention are described hereinbelow.

Amino acid residue linkers are usually at least one residue and can be 40 or more residues, more often 1 to 10 residues, but do not form αᵥβ₅ ligand epitopes. Typical amino acid residues used for linking are tyrosine, cysteine, lysine, glutamic and aspartic acid, or the like. In addition, a subject polypeptide can differ, unless otherwise specified, from the natural sequence of an αᵥβ₅ ligand by the sequence being modified by terminal-NH₂ acylation, e.g., acetylation, or thioglycolic acid amidation, by terminal-carboxylamidation, e.g., with ammonia, methylamine, and the like terminal modifications. Terminal modifications are useful, as is well known, to reduce susceptibility by proteinase digestion, and therefore serve to prolong half life of the polypeptides in solutions, particularly biological fluids where proteases may be present. In this regard, polypeptide cyclization is also a useful terminal modification, and is particularly preferred also because of the stable structures formed by cyclization and in view of the biological activities observed for such cyclic peptides as described herein.

Any peptide of the present invention may be used in the form of a pharmaceutically acceptable salt. Suitable acids which are capable of forming salts with the peptides of the present invention include inorganic acids such as trifluoroacetic acid (TFA) hydrochloric acid (HCI), hydrobromic acid, perchloric acid, nitric acid, thiocyanic acid, sulfuric acid, phosphoric acetic acid, propionic acid, glycolic acid, lactic acid, pyruvic acid, oxalic acid, malonic acid, succinic acid, maleic acid, fumaric acid, anthranilic acid, cinnamic acid, naphthalene sulfonic acid, sulfanilic acid or the like. HCl salt is particularly preferred.

Suitable bases capable of forming salts with the peptides of the present invention include inorganic bases such as sodium hydroxide, ammonium hydroxide, potassium hydroxide and the like; and organic bases such as mono-, di- and tri-alkyl and aryl amines (e.g. triethylamine, diisopropyl amine, methyl amine, dimethyl amine and the like) and optionally substituted ethanolamines (e.g. ethanolamine, diethanolamine and the like).

A peptide of the present invention also referred to herein as a subject polypeptide, can be synthesized by any of the techniques that are known to those skilled in the polypeptide art, including recombinant DNA techniques. Synthetic chemistry techniques, such as a solid-phase Merrifield-type synthesis, are preferred for reasons of purity, antigenic specificity, freedom from undesired side products, ease of production and the like. An excellent summary of the many techniques available can be found in Steward et al., "Solid Phase Peptide Synthesis", W.H. Freeman Co., San Francisco, 1969; Bodanszky, et al., "Peptide Synthesis", John Wiley & Sons, Second Edition, 1976; J. Meienhofer, "Hormonal Proteins and Peptides", Vol. 2, p. 46, Academic Press (New York), 1983; Merrifield, Adv. Enzymol., 32:221-96, 1969; Fields et al., Int. J. Peptide Protein Res., 35:161-214, 1990; and United States Patent No. 4,244,946 for solid phase peptide synthesis, and Schroder et al., "The Peptides", Vol. 1, Academic Press (New York), 1965 for classical solution synthesis, each of which is incorporated herein by reference. Appropriate protective groups usable in such synthesis are described in the above texts and in J.F.W. McOmie, "Protective Groups in Organic Chemistry", Plenum Press, New York, 1973.

In general, the solid-phase synthesis methods contemplated comprise the sequential addition of one or more amino acid residues or suitably protected amino acid residues to a growing peptide chain. Normally, either the amino or carboxyl group of the first amino acid residue is protected by a suitable, selectively removable protecting group. A different, selectively removable protecting group is utilized for amino acids containing a reactive side group such as lysine.

Using a solid phase synthesis as exemplary, the protected or derivatized amino acid is attached to an inert solid support through its unprotected carboxyl or amino group. The protecting group of the amino or carboxyl group is then selectively removed and the next amino acid in the sequence having the complimentary (amino or carboxyl) group suitably protected is admixed and reacted under conditions suitable for forming the amide linkage with the residue already attached to the solid support. The protecting group of the amino or carboxyl group is then removed from this newly added amino acid residue, and the next amino acid (suitably protected) is then added, and so forth. After all the desired amino acids have been linked in the proper sequence, any remaining terminal and side group protecting groups (and solid support) are removed sequentially or concurrently to generate the final linear polypeptide.

The resultant linear polypeptides prepared, for example, as described above may be reacted to form their corresponding cyclic peptides. An exemplary method for cyclizing peptides is described by Zimmer et al., Peptides 1992, pp. 393-394, ESCOM Science Publishers, B.V., 1993. Typically, tertbutoxycarbonyl protected peptide methyl ester is dissolved in methanol and sodium hydroxide solution are added and the admixture is reacted at 20°C (20C) to hydrolytically remove the methyl ester protecting group. After evaporating the solvent, the tertbutoxycarbonyl protected peptide is extracted with ethyl acetate from acidified aqueous solvent. The tertbutoxycarbonyl protecting group is then removed under mildly acidic conditions in dioxane cosolvent. The unprotected linear peptide with free amino and carboxy termini so obtained is converted to its corresponding cyclic peptide by reacting a dilute solution of the linear peptide, in a mixture of dichloromethane and dimethylformamide, with dicyclohexylcarbodiimide in the presence of 1-hydroxybenzotriazole and N-methylmorpholine. The resultant cyclic peptide is then purified by chromatography.

A particularly preferred cyclic peptide synthesis method is described by Gurrath et al., Eur. J. Biochem., 210:911-921 (1992), and described in the Examples. Particularly preferred peptides for use in the present methods in tissues primarily exhibiting αᵥβ₅-associated angiogenesis are described in the Examples, and include the polypeptides shown in SEQ ID NOs 4, 6, 7, 8 and 9.

### F. Methods For Identifying Antagonists of αᵥβ₅

The invention also describes assay methods for identifying candidate αᵥβ₅ antagonists for use according to the present methods. In these assay methods candidate molecules are evaluated for their potency in inhibiting αᵥβ₅ binding to natural ligands, and furthermore are evaluated for their potency in inhibiting angiogenesis in a tissue.

The first assay measures angiogenesis in the chick chorioallantoic membrane (CAM) and is referred to as the CAM assay. The CAM assay has been described in detail by others, and further has been used to measure both angiogenesis and neovascularization of tumor tissues. See Ausprunk et al., Am. J. Pathol., 79:597-618 (1975) and Ossonski et al., Cancer Res., 40:2300-2309 (1980).

The CAM assay is a well recognized assay model for in vivo angiogenesis because neovascularization of whole tissue is occurring. Actual chick embryo blood vessels are growing into the CAM or into the tissue grown on the CAM.

As demonstrated herein, the CAM assay illustrates inhibition of neovascularization based on both the amount and extent of new vessel growth. Furthermore, it is easy to monitor the growth of any tissue transplanted upon the CAM, such as a tumor tissue. Finally, the assay is particularly useful because there is an internal control for toxicity in the assay system. The chick embryo is exposed to any test reagent. As such, the health of the embryo is an indication of toxicity.

The second assay that measures angiogenesis is the in vivo rabbit eye model and is referred to as the rabbit eye assay. The rabbit eye assay has been described in detail by others and further has been used to measure both angiogenesis and neovascularization in the presence of angiogenic inhibitors such as thalidomide. See D'Amato, et al., Proc. Natl. Acad. Sci.. USA, 91:4082-4085 (1994).

The rabbit eye assay is a well recognized assay model for in vivo angiogenesis because the neovascularization process, exemplified by rabbit blood vessels growing from the rim of the cornea into the cornea, is easily visualized through the naturally transparent cornea of the eye. Additionally, both the extent and the amount of stimulation or inhibition of neovascularization or regression of neovascularization can easily be monitored over time.

Finally, the rabbit is exposed to any test reagent and as such the health of the rabbit is an indication of toxicity of the test reagent.

The third assay measures inhibition of direct binding of the natural ligand, vitronectin, to αᵥβ₅, and a preferred embodiment is described in detail in the Examples. The assay typically measures the degree of inhibition of binding of a natural ligand, such as vitronectin, to isolated αᵥβ₅ in the solid phase by ELISA, the inhibition of which is mediated by an αᵥβ₅-specific inhibition.

Thus, the assay can also be used to identify compounds which exhibit specificity for αᵥβ₅ and do not inhibit natural ligands from binding other integrins. The specificity assay is conducted by running parallel ELISA assays where both αᵥβ₅ and other integrins are screened concurrently in separate assay chambers for their respective abilities to bind a natural ligand and for the candidate compound to inhibit the respective abilities of the integrins to bind a preselected ligand. Preferred screening assay formats are described in the Examples.

### Examples

The following examples relating to this invention are illustrative and should not, of course, be construed as specifically limiting the invention. Moreover, such variations of the invention, now known or later developed, which would be within the purview of one skilled in the art are to be considered to fall within the scope of the present invention hereinafter claimed.

### 1. Preparation of αᵥβ₅-Specific Monoclonal Antibodies

The monoclonal antibodies, P1F6 and P5H9, were produced using standard hybridoma methods by immunization into RBF/DnJ mice with A549 lung carcinoma cells as described by Wayner et al., J. Cell Biol., 113:919-929 (1991), the disclosure of which is hereby incorporated by reference. Spleens were removed from the immunized mice and fused with Ns-1/FOX-NY myeloma cells. Hybridomas producing antibody directed to carcinoma cell vitronectin receptors were screened by the specific inhibition of UCLA-P3 adhesion to vitronectin-coated surfaces as described by Wayner et al. and cloned by limiting dilution on thymocyte feeder layers.

Both the P1F6 and P5H9 monoclonal antibodies have been shown to specifically immunoreact with the αᵥβ₅ complex, and not immunoreact with αᵥ subunit, with β₅ subunit, or with other integrins. The P1F6 monoclonal antibody is commercially available from Gibco BRL (Life Technologies, Inc., Gaithersburg, MD) and the P5H9 monoclonal is available from Dr. E. Wayner at the Fred Hutchinson Cancer Research Institute, Seattle, WA.

Other αᵥβ₅ monoclonal antibodies for use in this invention are similarly derived and characterized as described herein. In addition, αᵥβ₅ monoclonal antibodies are produced by fusing spleens isolated from mice that receive immunizations with the αᵥβ₅ receptor in either an impure or purified form. Purification of the αᵥβ₅ is a procedure well known to one of ordinary skill in the art of integrin biology and has also been described by Smith et al., J. Biol. Chem., 265:11008-11013 (1990), the disclosure of which is hereby incorporated by reference. Once purified, the isolated receptor is prepared as an immunogen for immunizing mice as described in Section E2 and as prepared essentially as described by Kohler and Milstein, Nature, 256:495-497 (1975), the disclosure of which is hereby incorporated by reference. The resultant hybridoma clones are screened for reactivity with the immunogen and are then characterized as described in the following Examples.

### 2. Characterization of the Specificity of the Anti-αᵥβ₅ Monoclonal Antibodies and Use in Mapping the Tissue Distribution of αᵥβ₅ Expression

### A. Specificity for Vitronectin

The P5H9 monoclonal antibody prepared in Example 1 was shown by Wayner et al., J. Cell. Biol., 113:919-929 (1991) to block attachment of UCLA-P3 carcinoma cells to vitronectin while not affecting cell attachment to collagen or fibronectin. The same cells were also shown to contain only the αᵥβ₅ vitronectin receptor and not one with αᵥβ₃ specificity, immunoprecipitating a heterodimer consisting of an α chain (160 kD) and a β chain (95 kD) with nonreducing conditions. The αᵥβ₅ receptor detected by P5H9 was also shown to mediate adhesion of M21 melanoma cells and H2981 carcinoma cells to vitronectin. The P1F6 monoclonal antibody has the same immunoreactivity profile.

### B. Immunofluorescence with Anti-Intearin Receptor Antibodies

During wound healing, the basement membranes of blood vessels express several adhesive proteins, including von Willebrand factor, fibronectin, and fibrin. In addition, several members of the integrin family of adhesion receptors are expressed on the surface of cultured smooth muscle and endothelial cells. See, Cheresh, Proc. Natl. Acad. Sci., USA, 84:6471 (1987); Janat et al., J. Cell Physiol., 151:588 (1992); and Cheng et al., J. Cell Physiol., 139:275 (1989).

In addition to the structure and function of the integrin β₅ subunit, the tissue distribution of the subunit by mapping with other anti-β₅ monoclonal antibodies has been described by Pasqualini et al., J. Cell Sci., 105:101-111 (1993), the disclosure of which is hereby incorporated by reference.

The β₅ subunit-specific monoclonal antibodies described above, similar to those described in Example 1, were secreted from hybridomas that were prepared using splenocytes from a mouse that received immunizations with the A549 human lung carcinoma cell line. The hybridomas were selected by positive surface staining of A549 cells with the hybridomas culture supernatant and by immunoprecipitation of αᵥβ₅ complexes from surface-labeled A549 extracts. The monoclonal antibodies were then used to map the tissue distribution of the β₅ subunit in normal human thymus, skin and kidney. Four micron thick sections were cut from the frozen tissue blocks on a cryostat microtome for subsequent streptavidin-biotin immunoperoxidase staining with antibodies specific for the β₅ integrins performed as described in the Pasqualini et al. reference.

Staining of thymic sections showed the distribution of β₅ on blood vessels, Hassal's corpuscles, cortical and medullary stromal cells, and basement membranes. Skin sections showed β₅ on the basal layer of the epidermis and on some dermal blood vessel walls, and kidney sections showed staining of glomerular regions, juxtaglomerular apparatus, proximal convoluted tubules and collecting tubules. Thus, the distribution of β₅ is heterogeneous to different cell types including and, more importantly, on capillary endothelial cells, the staining of which was consistent with staining of cultured umbilical vein endothelial cells.

### C. Immunofluorescence of Human Retinal Tissue from Patients with Ocular Disease with Anti-Integrin Receptor Antibodies

Ocular neovascularization is the most common pathological change observed in the vast majority of eye diseases that result in catastrophic loss of vision. The growth of new blood vessels from the pre-existing choroidal, retinal or paralimbal vessels can lead to edema, hemorrhage or fibrovascular membrane formation resulting in disruption of the normal anatomic relationships of the eye and concomitant loss of normal visual function.

Under physiological conditions, angiogenesis is highly regulated and has been shown to be activated by specific angiogenic cytokines such as basic fibroblast growth factor (bFGF) and tumor necrosis factor-α (TNF-α). As described by Brooks et al., Science, 264:569-571 (1994), monoclonal antibodies against αᵥβ₃ have been shown to be block both bFGF- and TNF-α-induced angiogenesis in model systems including the CAM model described below. As described in Examples 4-6, monoclonal antibodies against αᵥβ₅ block a separate pathway of angiogenesis, specifically that induced by vascular endothelial growth factor (VEGF), transforming growth factor-α (TGF-α) and epidermal growth factor (EGF).

Thus, as described herein in the context of the present invention, two pathways of angiogenesis are defined by distinct integrins, αᵥβ₃ and αᵥβ₅. To investigate the expression and role of these integrins in human ocular disease, epiretinal neovascular membranes and subretinal neovascular membranes were obtained en bloc at vitrectomy from patients with proliferative diabetic retinopathy (PDR). These patients had been followed clinically and were selected for histological evaluation on the basis of having active, proliferative neovascular disease documented by clinical examination and fundus fluorescein angiography. The obtained tissue was frozen immediately in Tissue Tek cryopreservative and sectioned.

When the tissues from these patients were examined by immunofluorescence, the blood vessels were positive for the integrin αᵥβ₃ as indicated by immunoreactivity with the mouse monoclonal antibody LM609. The distribution of the integrin appeared to be restricted to blood vessels and coincided with staining for a marker of blood vessels, von Willebrand Factor, as mapped with a rabbit antibody to the factor. The sites of immunoreactivity were visualized with either rhodamine-conjugated anti-mouse immunoglobulin or fluorescein-conjugated anti-rabbit immunoglobulin, the use of both of which allowed co-localization of the integrin location and blood vessel-specific antibodies.

Specimens obtained from normal eyes or patients with atrophic membranes free from actively proliferating blood vessels were negative for the integrin αᵥβ₃ by immunofluorescence.

In parallel, the same tissues were analyzed immunohistochemically for the presence and distribution of αᵥβ₅ with the anti-αᵥβ₅ monoclonal antibody, P1F6, prepared in Example 1. The staining revealed that αᵥβ₅ was present on blood vessels that co-localized with the distribution of von Willebrand factor. However, the non-vascular tissue also displayed limited fluorescence with the P1F6 antibody indicating a wider distribution of αᵥβ₅. This was in contrast to the presence of αᵥβ₃ that was limited to blood vessels.

When immunofluorescent staining of membranes was compared between αᵥβ₃ and αᵥβ₅ with the respective antibodies LM609 and P1F6, the pattern of staining on the blood vessel wall was virtually identical indicating that both αᵥβ₃ and αᵥβ₅ are displayed on the surface of newly proliferating human blood vessels present in neovascular eye diseases such as diabetic retinopathy.

The results described herein thus show that the αᵥβ₅ integrin receptor is selectively expressed in specific tissue types in which angiogenesis is occurring, such as that seen with neovascular membranes from patients having active, proliferative neovascular disease. These tissues, along with those tissues exposed to particular growth factors as described below in Examples 4-6, therefore provide ideal targets for therapeutic aspects of this invention.

### 3. Preparation of Synthetic Peptides

The cyclic polypeptides used in practicing the methods of this invention were synthesized using standard solid-phase synthesis techniques as, for example, described by Merrifield, Adv. Enzymol., 32:221-296 (1969), and Fields, G.B. and Noble, R.L., Int. J. Peptide Protein Res., 35:161-214 (1990).

Two grams (g) of BOC-Arg-Gly-Asp-D-Phe-Val-OMe (SEQ ID NO 1) were first dissolved in 60 milliliters (ml) of methanol to which was added 1.5 ml of 2 N sodium hydroxide solution to form an admixture. The admixture was then stirred for 3 hours at 20 degrees C (20C). After evaporation, the residue was taken up in water and acidified to pH 3 with diluted HCl and extracted with ethyl acetate. The extract was dried over Na₂SO₄, evaporated again and the resultant BOC-Arg-Gly-Asp-D-Phe-Val-OH (SEQ ID NO 2) was stirred at 20C for 2 hours with 20 ml of 2 N HCl in dioxane. The resultant admixture was evaporated to obtain H-Arg-Gly-Asp-D-Phe-Val-OH (SEQ ID NO 3) that was subsequently dissolved in a mixture of 1800 ml of dichloromethane and 200 ml of dimethylformamide (DMF) followed by cooling to 0C. Thereafter, 0.5 g of dicyclohexylcarbodiimide (DCCI), 0.3 g of 1-hydroxybenzotriazole (HOBt) and 0.23 ml of N-methylmorpholine were added sequentially with stirring.

The resultant admixture was stirred for another 24 hours at 0C and then at 20C for yet another 48 hours. The solution was concentrated and treated with a mixed bed ion exchanger to remove salts. After the resulting resin was removed by filtration, the clarified solution was evaporated and the residue was purified by chromatography resulting in the recovery of cyclo(Arg-Gly-Asp-D-Phe-Val) (also listed in single letter code as c-RGDfV) (SEQ ID NO 4). The lower case letters in the peptide indicate the D form of the amino acid and not the L form as indicated by capital letters.

The cyclic control peptide, cyclo(Arg-Ala-Asp-D-Phe-Val) (also listed in single letter code as RADfV) (SEQ ID NO 5) was prepared as described above. The cyclic peptide c-RADfV (SEQ ID NO 5) has previously been shown to inhibit binding of fibrinogen to the integrin αᵥβ₃, and not inhibit binding of fibrinogen to the integrins α_{IIb}β₃ or α₅β₁ (Pfaff, et al., J. Biol . Chem., 269:20233-20238, 1994).

Other peptides that are specifically inhibitory to the binding of natural ligands to αᵥβ₅ are similarly prepared as tested for specificity and range of activity as described in the following examples. These include the following peptides that were analogously obtained: cyclo(Gly-D-Arg-Gly-Asp-Phe-Val) (SEQ ID NO 6) and cyclo(Arg-Gly-Asp-Phe-D-Val) (SEQ ID NO 7). The peptide having the amino acid residue sequence Tyr-Thr-Ala-Glu-Cys-Lys-Pro-Gln-Val-Thr-Arg-Gly-Asp-Val-Phe (SEQ ID NO 8) was also synthetically prepared.

### 4. Inhibition of Growth Factor-Induced Angiogenesis With αᵥβ₅ Antagonists as Measured by In Vivo Rabbit Eye Model Assay

The effect of anti-αᵥβ₅ antagonists on growth factor-induced angiogenesis can be observed in naturally transparent structures as exemplified by the cornea of the eye. New blood vessels grow from the rim of the cornea, which has a rich blood supply, toward the center of the cornea, which normally does not have a blood vessels. Stimulators of angiogenesis, such as VEGF and TGF-α, when applied to the cornea induce the growth of new blood vessels from the rim of the cornea. Antagonists of angiogenesis, applied to the cornea, inhibit the growth of new blood vessels from the rim of the cornea. Thus, the cornea undergoes angiogenesis through an invasion of endothelial cells from the rim of the cornea into the tough collagen-packed corneal tissue which is easily visible. The rabbit eye model assay therefore provides an in vivo model for the direct observation of stimulation and inhibition of angiogenesis following the implantation of compounds directly into the cornea of the eye.

### A. In Vivo Rabbit Eye Model Assay

### 1) Angiogenesis Induced by Growth Factors

Angiogenesis was induced in the in vivo rabbit eye model assay with growth factors and is described in the following.

### a. Preparation of Hydron Pellets Containing Growth Factor and Monoclonal Antibodies

Hydron polymer pellets containing growth factor and monoclonal antibodies (mAbs) were prepared as described by D'Amato, et al., Proc. Natl. Acad. Sci., 91:4082-4085 (1994). The individual pellets contained 750 ng of the growth factor (also referred to as cytokine), specifically either bFGF or VEGF, bound to sucralfate (carafate) (Carafet, Marion Merrell Dow Corporation, Cincinnati, OH) to stabilize the cytokines and ensure their slow release into the surrounding tissue. In addition, hydron pellets were prepared which contained either 40 µg of the mAb P1F6 (anti-αᵥβ₅) or the control antibody, LM609 (anti-αᵥβ₃), in PBS.

All of the mAbs tested were purified from ascites fluid using Protein-A Sepharose CL-4B affinity column chromatography according to well-known methods. The eluted immunoglobulin was then dialyzed against PBS and treated with Detoxi-gel (Pierce Chemicals, Rockford, IL) to remove endotoxin. Endotoxin has been shown to be a potent angiogenic and inflammatory stimulant. Monoclonal antibodies were therefore tested for the presence of endotoxin with the Chromogenic Limulus Amebocyte Lysate Assay (BioWhittaker, Walkersville, MD) and only those mAbs without detectable endotoxin were used in the rabbit eye model assay.

The pellets were cast in specially prepared Teflon pegs that had a 2.5 mm core drilled into their surfaces. Approximately 12 µl of casting material was placed into each peg and polymerized overnight in a sterile hood. Pellets were then sterilized by ultraviolet irradiation.

A series of eight animals were used for paired eye experiments where each animal received a Hydron implant containing a preselected cytokine with a preselected antibody or control immunoglobulin. Specifically, for each rabbit, one cornea was surgically implanted with a Hydron pellet containing either bFGF or VEGF in conjunction with mAb P1F6 and the other cornea was treated with either bFGF or VEGF in conjunction with MAb LM609. Individual pellets were implanted into surgically created "pockets" formed in the mid-stroma of the cornea of rabbits. The surgical procedure was done under sterile technique using a Wild model M691 operating microscope equipped with a beamsplitter to which was mounted a camera for photographically recording individual corneas. A 3 mm by 5 mm "pocket" was created in the corneal stroma by making a 3 mm incision to half the corneal thickness with a 69 Beaver blade. The stroma was dissected peripherally using an iris spatula and the pellet was implanted with its peripheral margin 2 mm from the limbus.

During the following 12 days, the cytokines and mAbs diffused from the implanted pellets into the surrounding tissue thereby effecting angiogenesis from the rim of the cornea.

The left and right corneas are respectively referred to as OS and OD. The corneas were then observed for 12 days. Photographs were taken on postoperative day 10, the time at which neovascularization is maximal.

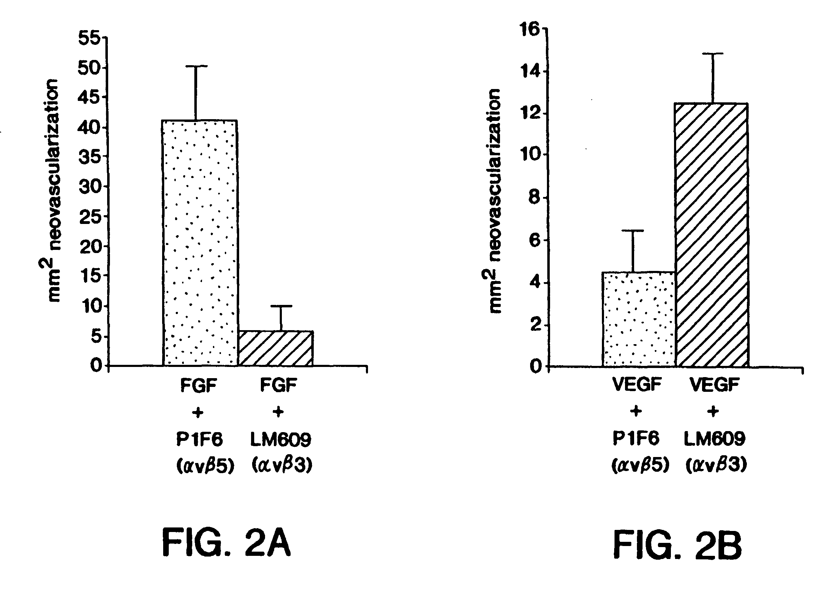

Representative photographic results of the above-treatments with cytokine/mAb admixtures are shown in Figures 1AID. The parallel quantitation of mAb inhibition of cytokine-induced angiogenesis is shown in Figures 2A and 2B. In Figures 1A and 1D, in which corneas were respectively exposed to bFGF/PIF6 and VEGF/LM609 combinations, cytokine-induced angiogenesis with edema is prominent as indicated by the large arrows. Therefore, the αᵥβ₅ antibody, P1F6, was not effective at inhibiting bFGF-induced angiogenesis. Similarly, the αᵥβ₃ antibody, LM609, was not effective at inhibiting VEGF-induced angiogenesis.

In contrast, when the cytokine/mAb combinations of bFGF/LM609 and VEGF/P1F6 were used in the rabbit model, the cytokine-induced angiogenesis was inhibited by the antibodies as shown in Figures 1B and 1C, respectively. In these figures, normal conjunctival limbal vessels indicated by the small arrows are shown indicating effectiveness of the integrin antibodies in inhibiting one type of cytokine-induced angiogenesis.

The effects of specific mAb integrin immunoreactivity on the above cytokine-induced angiogenesis is also quantified as shown in Figures 2A and 2B. Angiogenesis was stimulated with either bFGF or VEGF as shown respectively in Figures 2A and 2B. The treated eyes were photographed daily through a Wild operating microscope outfitted with a Nikon camera. Photographs were recorded on Kodak Ektachrome 64T slide film and images were converted for computer-assisted quantitation using Biorad's Molecular Analyst 1.1 software after acquisition through a Model GS670 imaging densitometer. Histograms illustrating the mean neovascular area +/- the standard error (n=8 for each of two series) after exposure to the mAbs P1F6 or LM609.

As shown in Figure 2A, LM609 reduced bFGF-induced angiogenesis by 86% (p <0.005, paired t-test) when compared to treatment of the paired eye on the same animal with P1F6. When VEGF was used to stimulate angiogenesis as shown in Figure 2B, the opposite effect was observed where P1F6 reduced the mean area of neovascularization by 60% (p <0.03, paired t-test) compared to the LM609-treated eye that had a minimal effect on VEGF-induced angiogenesis.

Significantly, only the newly cytokine-induced blood vessels were effected by exposure to a particular mAb while the pre-existing perilimbal vessels were unaffected by either mAb suggesting that the effects observed are restricted to newly forming blood vessels of the cornea.

Similar assays are performed with synthetic peptides prepared in Example 3 and as described below for use in inhibiting cytokine-induced angiogenesis that is specifically correlated with αᵥβ₅ expression.

To confirm these results indicating that angiogenesis induced by a particular cytokine was only effected by one type of anti-integrin antibody, specifically that αᵥβ₅ integrin receptor plays a role in VEGF-induced angiogenesis, another neovascular model of the chick chorioallantoic membrane (CAM) was evaluated with the combinations of cytokines and integrin antibodies as shown in the next Example.

### 5. Angiogenesis in the Chick chorioallantoic Membrane (CAM) Preparation

### A. Characterization of the Untreated CAM

### 1) Preparation of the CAM

Angiogenesis can be induced on the chick chorioallantoic membrane (CAM) after normal embryonic angiogenesis has resulted in the formation of mature blood vessels. Angiogenesis has been shown to be induced in response to specific cytokines or tumor fragments as described by Leibovich et al., Nature, 329:630 (1987) and Ausprunk et al., Am. J. Pathol., 79:597 (1975). CAMs were prepared from chick embryos for subsequent induction of angiogenesis and inhibition thereof as described below and in Example 6 with the αᵥβ₅ antagonists of this invention.

Ten day old chick embryos were obtained from McIntyre Poultry (Lakeside, CA) and incubated at 37C with 60% humidity. A small hole was made through the shell at the end of the egg directly over the air sac with the use of a small crafts drill (Dremel, Division of Emerson Electric Co., Racine, WI). A second hole was drilled on the broad side of the egg in a region devoid of embryonic blood vessels determined previously by candling the egg. Negative pressure was applied to the original hole, which resulted in the CAM (chorioallantoic membrane) pulling away from the shell membrane and creating a false air sac over the CAM. A 1.0 centimeter (cm) x 1.0 cm square window was cut through the shell over the dropped CAM with the use of a small model grinding wheel (Dremel). The small window allowed direct access to the underlying CAM.

The resultant CAM preparation was then used at 10 days of embryogenesis where angiogenesis has subsided. The preparation was thus used in this invention for inducing renewed angiogenesis in response to cytokine treatment.

### 2) Histology of the CAM

To analyze the microscopic structure of the chick embryo CAMs, six micron (µm) thick sections were cut from the frozen blocks on a cryostat microtome for immunofluorescence analysis.

Typical of an untreated 10 day old CAM is an area devoid of blood vessels. As angiogenesis in the CAM system is subsiding by this stage of embryogenesis, the system is useful in this invention for stimulating with various cytokines the production of new vasculature from existing vessels from adjacent areas into areas of the CAM currently lacking any vessels.

As shown in the CAM model and in the following Examples, while the blood vessels are undergoing new growth in normal embryogenesis or induced by cytokines, the blood vessels ' are expressing αᵥβ₃ and αᵥβ₅.

### B. Angioaenesis Induced by Growth Factors

Angiogenesis has been shown to be induced by cytokines or growth factors as described in Example 4A in the rabbit eye model. In the experiments described herein, angiogenesis in the rabbit corneal preparation described in Example 4 was similarly induced by growth factors that were topically applied onto the CAM blood vessels as described herein.

Angiogenesis was induced by placing a 5 millimeter (mm) X 5 mm Whatman filter disk (Whatman Filter paper No. 1) saturated with Hanks Balanced Salt Solution (HBSS, GIBCO, Grand Island, NY) or HBSS containing preselected cytokines at a preselected concentration, i.e, one to test the effect on angiogenesis, on the CAM of a 10 day chick embryo in a region devoid of blood vessels and the windows were later sealed with tape. Angiogenesis was monitored by photomicroscopy after 72 hours. CAMs were snap frozen then 6 µm cryostat sections were fixed with acetone and stained by immunofluorescence as described in Example 2B and 2C with 10 µg/ml of selected anti-integrin antibodies, including those directed against αᵥβ₅ as described in Example 1.

Previous studies by Brooks et al., Science, 264:569-571 (1994), have shown that blood vessels are readily apparent in both the bFGF and TNF-α treated preparations but are not present in the untreated CAM. The authors have also shown that αᵥβ₃ expression was enhanced following bFGF-induced angiogenesis. While the expression of integrin β₁ did not change from that seen in an untreated CAM, β₁ was also readily detectable on stimulated blood vessels.

These published findings indicated that in both human and chick, blood vessels involved in angiogenesis show enhanced expression of αᵥβ₃. Consistent with this, expression of αᵥβ₃ on cultured endothelial cells were induced by various cytokines in vitro as described by Janat et al., J. Cell Physiol., 151:588 (1992); Enenstein et al., Exp. Cell Res., 203:499 (1992) and Swerlick et al., J. Invest. Derm., 99:715 (1993).

In this invention, a separate cytokine-mediated pathway for simulating angiogenesis that is dependent upon expression and activation of a different adhesive integrin receptor, αᵥβ₅, has now been determined. The effect of exposure of a CAM as described herein to the cytokines VEGF, TGF-α and EGF in relationship to the expression of αᵥβ₅, to angiogenesis and inhibition thereof with αᵥβ₅ antagonists is described in Example 6.

### C. Angiogenesis Induced by Tumors

To investigate the role of αᵥβ₅ in tumor-induced angiogenesis, various αᵥβ₅-negative human melanoma and carcinoma fragments are used in the CAM assay that are previously grown and isolated from the CAM of 17 day chick embryo as described by Brooks et al., J. Cell Biol., 122:1351 (1993) and as described herein.

Angiogenesis is induced in the CAM assay system by direct apposition of a tumor fragment on the CAM. Preparation of the chick embryo CAM is identical to the procedure described above. Instead of a filter paper disk, a 50 milligram (mg) to 55 mg in weight fragment of one αᵥβ₅-negative tumor resulting from growth of cell line suspensions described below, is placed on the CAM in an area originally devoid of blood vessels.

The cell lines, rabdomyosarcoma, myeloid (HL-60 or KG-1), and lymphoid (T cells - Jurkat, HPB/ALL, PEER; and various B cell lines) as described by Pasqualini et al. J. Cell Sci., 105:101-111 (1993), are used to grow the solid human tumors on the CAMs of chick embryos. A single cell suspension of the various cell lines are first applied to the CAMs in a total volume of 30 µl of sterile HBSS. The windows are sealed with tape and the embryos are incubated for 7 days to allow growth of human tumor lesions. At the end of 7 days, now a 17 day embryo, the tumors are resected from the CAMs and trimmed free of surrounding CAM tissue. The tumors are sliced into 50 mg to 55 mg tumor fragments for use in angiogenesis. The tumor fragments are placed on a new set of 10 day chick embryo CAMs as described in Example 5A in an area devoid of blood vessels.

Tumors grown in vivo on the chick embryo CAMs with and without topical or intravenous application of αᵥβ₅-inducing cytokines (VEGF, TGF-α, or EGF) are then stained for αᵥβ₅ expression with mAbs, P1F6 or P5H9, as previously described.

These CAM tumor preparations are then subsequently treated as described in Examples 6C and 6D for measuring the effects of antibodies and peptides on tumor-induced angiogenesis.

In one embodiment, hamster melanoma cells, CS-1, obtained from Dr. Caroline Damsky from University of California at San Francisco, were used in the CAM assay as described above for formation of melanoma tumors. Following the transfer of approximately a 50 mg CS-1 tumor fragment on a new 10 day chick embryo CAM, separate preparations received an intravenous injections of either 100 µg or 300 µg of P1F6 antibody, LM609 antibody or control CSAT (anti-β1) antibody. An additional control included a preparation that received no treatment. The results are discussed below in Example 6D.

### 6. Inhibition of Angiogenesis as Measured in the CAM Assay

### A. Inhibition of Growth Factor-Induced Angiogenesis by Intravenous Application of Inhibitors

The effect on growth factor-induced angiogenesis with monoclonal antibodies intravenously injected into the CAM preparation was evaluated for use as an in vivo model system of this invention.

Following active neovascularization, once the vessels have stopped developing, the expression of αᵥβ₅ diminishes to levels not detectable by immunofluorescence analysis. This regulation of αᵥβ₅ expression in blood vessels undergoing angiogenesis as contrasted to the lack of expression in mature vessels provides for the unique ability of this invention to control and inhibit angiogenesis as shown below as modeled in the CAM angiogenesis assay system.

The preparation of the chick embryo CAMs for intravenous injections was essentially as described above.

Angiogenesis was first induced on 10 day old chick embryos by application of growth factor-saturated filter disks. Specifically, in the first assays, angiogenesis was induced by exposure to either bFGF or VEGF, each at a concentration of 150 ng/ml.

For application of growth factors, during the candling procedures, prominent blood vessels were selected and marks were made on the egg shell to indicate their positions. The holes were drilled in the shell, the CAMs were dropped and growth factor-saturated filter papers were then separately placed on the CAMs as described above. The windows were sealed with sterile tape and the embryos were replaced in the incubator.

Twenty four hours later, a second small window was carefully cut on the lateral side of the egg shell directly over prominent blood vessels selected previously. The outer egg shell was carefully removed leaving the embryonic membranes intact. The shell membrane was made transparent with a small drop of mineral oil (Perkin-Elmer Corp, Norwalk, CT) which allowed the blood vessels to be visualized easily. Then, phosphate buffered saline (PBS), 75 µg of purified sterile anti-integrin antibodies or 75 µg of synthetic peptides (cyclic peptide RGDfV, SEQ ID NO 4 and control cyclic peptide RADfV, SEQ ID NO 5) in PBS were injected into blood vessels apparent on the growth factor-induced CAMs. The windows were sealed with tape and the embryos were allowed to incubate until 72 hours.

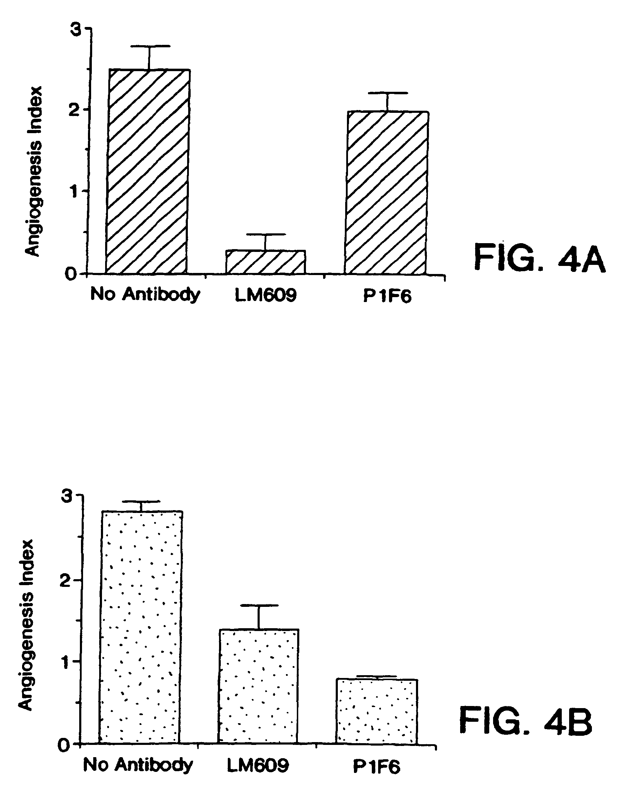

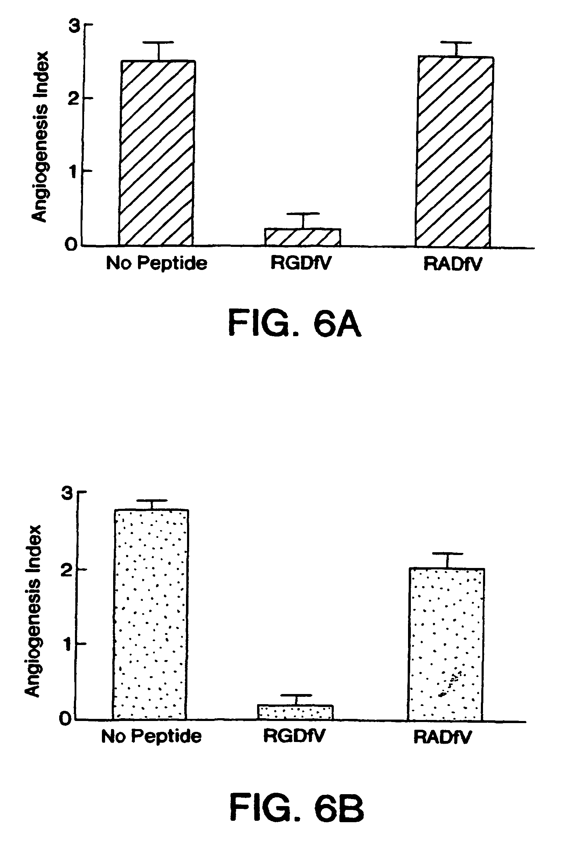

The filter discs and representative surrounding CAM tissues were photographed in a stereomicroscope (Figures 3A-3F and Figures 5A-5F) and the mean angiogenic index +/- the standard error was determined for 12 CAMs per condition (Figures 4A-4B and Figures 6A-6B). Angiogenesis was scored for each embryo in a double blind manner by analyzing the number and extent of branching of blood vessels within the area of each disc. The scores ranged from 1 (low) to 4 (high) and the angiogenesis index was determined by subtracting a background of 1 from all data.

Specificity of integrin antibody-mediated inhibition of growth factor-induced angiogenesis in the CAM model mirrored that seen in the rabbit cornea model described above. As respectively shown in Figures 3A and 3B, both bFGF and VEGF caused angiogenesis in the control PBS-treated CAM. Treatment with the αᵥβ₅-specific antibody, P1F6, however, resulted in inhibition of VEGF-induced angiogenesis as shown in Figure 3D while no inhibition was detected on bFGF-induced angiogenesis as seen in Figure 3C. In contrast, the LM609 αᵥβ₃-specific antibody inhibited bFGF-induced angiogenesis (Figure 3E) but had little effect on angiogenesis in the VEGF-induced CAM (Figure 3F).

These results are also shown in the bar graphs of Figures 4A and 4B, respectively for both bFGF- and VEGF-treated CAMs, in which the angiogenesis index is plotted against exposure to either LM609 or P1F6 along with no antibody exposure as a control. Thus, inhibition of growth factor-induced angiogenesis by integrin-specific antibodies is dependent upon the type of growth factor.

Exposure to RGD-containing peptides supports the above results. In the presence of PBS, as shown in Figures 5A and 5B, exposure to both bFGF and VEGF resulted in angiogenesis in the control CAM. In contrast, the cyclic peptide antagonist RGDfV (SEQ ID NO 4), directed to both αᵥβ₃ and αᵥβ₅, abolished angiogenesis induced by either bFGF or VEGF. The cyclic peptide RADfV (SEQ ID NO 5) did not effect angiogenesis in either the bFGF- or VEGF-treated CAM preparations. The results are also shown in Figures 6A and 6B where the angiogenesis index of bFGF- and VEGF-stimulated CAMS are graphed showing exposure to test and control peptides. Thus, these findings together with those in the rabbit corneas indicate that bFGF- and VEGF-induced angiogenesis depend on distinct but homologous αᵥ-specific integrins that however are both inhibitable with the cyclic peptide RGDfV.

Additional similar assays are performed with synthetic peptides prepared as described in Example 3 to define peptides that exhibit specificity to αᵥβ₅ and not αᵥβ₃ correlated angiogenesis. Assays are also performed with the organic molecules prepared as described in Example 10.

The specificity of integrin antibody-inhibition of growth factor-induced angiogenesis was further confirmed and strengthened by extending the growth factor angiogenesis induction analyses to include tumor necrosis factor-α (TNF-α), transforming growth factor-α (TGF-α) or the phorbol ester, 4-β-phorbol-12-myristate-13-acetate (PMA).

The above growth factors (cytokines), including bFGF and VEGF, were separately applied at a concentration of 1.0 µg/ml to the 10 day old CAM model as previously described. PMA was used at a concentration of 20 ng/ml.

After 24 hours after growth factor treatment, the antibodies, LM609 and P1F6, or the protein kinase C (PKC) inhibitor, calphostin C, were separately provided to the CAM model, either by a single intravascular dose as described above or by topical administration as described below in the next example. For intravascular injections over the next 3 day consecutive period, the antibodies were used at a concentration of 75 µg per embryo and the calphostin C was at a dosage of 100 nM.

On day 13, filter discs and associated CAM tissue were dissected and analyzed for angiogenesis with a stereo microscope. Angiogenesis was scored in a double blind manner by analyzing the number and extent of branching of the blood vessels within the area of the discs. The scores ranged from low (1) to high (4). The angiogenesis index was determined by subtracting a background score of 1 from all data. Experiments were repeated 2-4 times with 5-6 embryos per condition.

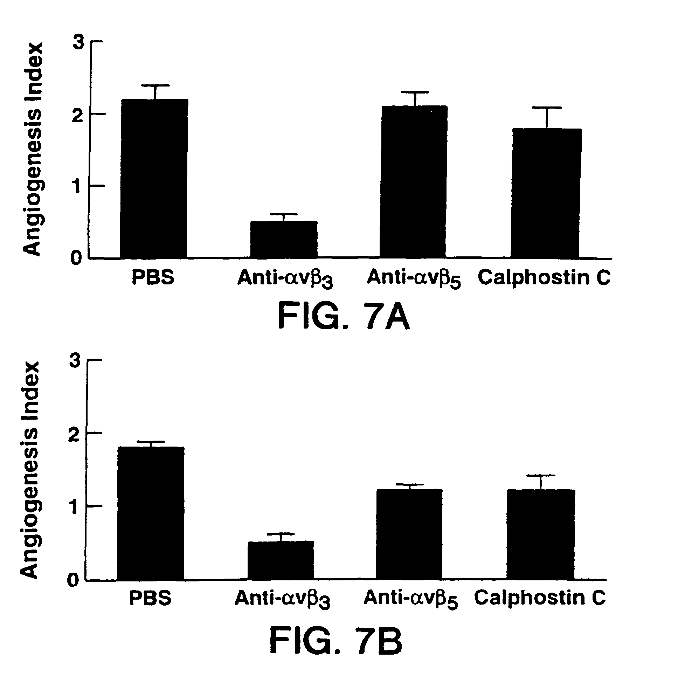

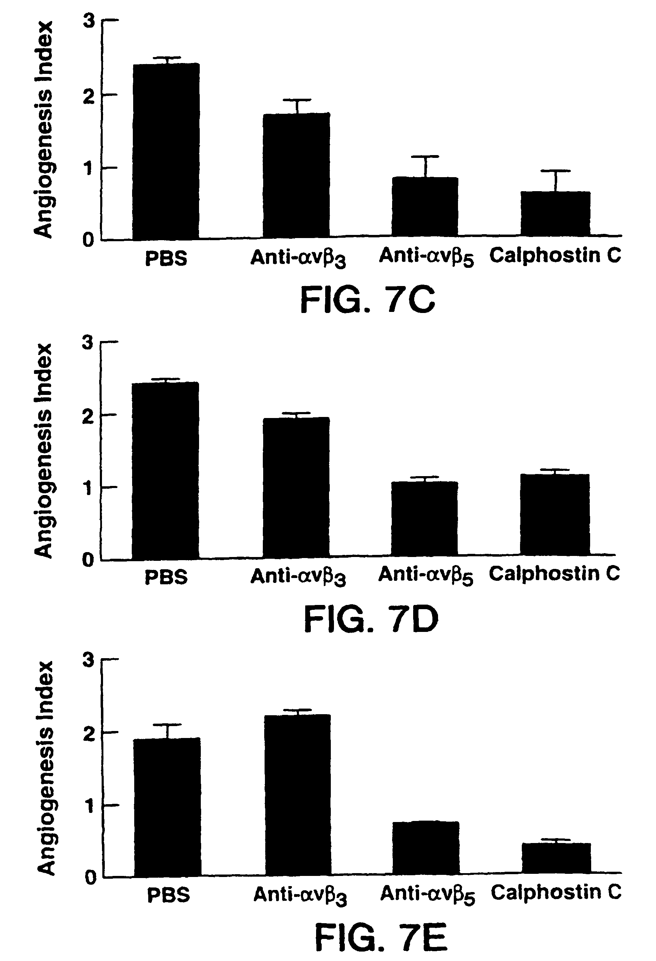

As shown respectively in Figures 7A and 7B, the anti-αᵥβ₃ antibody, LM609, blocked angiogenesis in response to bFGF and ' TNF-α whereas the anti-αᵥβ₅ antibody, P1F6, had little inhibitory effect. In contrast, as shown respectively in Figures 7C-7E, P1F6 was effective at inhibiting angiogenesis induced by VEGF, TGF-α, or PMA whereas LM609 failed to do so.

PMA, a potent inducer of angiogenesis, is capable of activating protein kinase C (PKC), an intracellular family of serine threonine kinases. Therefore, we also examined the effects of calphostin C, a PKC inhibitor, on angiogenesis on the chick CAM. Calphostin C blocked angiogenesis induced by PMA (Figure 7E) as well as VEGF and TGF-α (respectively shown in Figures 7C and 7D) while having minimal effects on bFGF- or TNF-α mediated angiogenesis (respectively shown in Figures 7A and 7B).

Together, these results indicate the existence of two separate distinct angiogenesis pathways where one is dependent upon an αᵥβ₃-mediated signal that is largely independent of PKC, as previously described by Brooks et al., Science, 264:569-571 (1994), and a second pathway is potentiated by an αᵥβ₅-mediated transduction signal that critically depends of PKC activation.

In addition to the above experiments, to determine the localization of the P1F6 and LM609 mAbs in CAM tissues that were inoculated intravenously with LM609, the fixed sections are blocked with 2.5% BSA in HBSS for 1 hour at room temperature followed by staining with a 1:250. dilution of goat anti-mouse rhodamine labeled secondary antibody (Tago). The sections are then analyzed with a Zeiss immunofluorescence compound microscope.

### B. Inhibition of Growth Factor-Induced Angiogenesis by Topical Application of Inhibitors

To determine whether αᵥβ₅ plays an active role in angiogenesis, filter disks saturated with growth factors described above are placed on CAMs to induce angiogenesis followed by application of either P1F6 or LM609.

Disks are then treated with 50 ml HBSS containing 25 mg of mAb in a total volume of 25 µl of sterile HBSS at 0, 24, and 48 hours. At 72 hours, CAMs are harvested and placed in a 35 mm petri dish and washed once with 1 ml of PBS. The bottom side of the filter paper and CAM tissue is then analyzed under an Olympus stereo microscope, with two observers in a double-blind fashion. Angiogenesis inhibition is considered significant when CAMs exhibits >50% reduction in blood vessel infiltration of the CAM directly under the disk. Experiments are repeated four times per antibody, with 6 to 7 embryos per condition.

To examine the effects of the integrin antibodies on preexisting mature blood vessels present from normal vessel development adjacent to the areas devoid of vessels, filter disks saturated with mAbs are placed on vascularized regions of CAMs from 10 day embryos that do not receive topical application of cytokine.

CAM assays are also performed with the synthetic peptides of this invention to determine the effect of cyclic and linearized peptides on growth factor induced angiogenesis. Eight µg of peptides, prepared as previously described, are separately presented in a total volume of 25 µl of sterile HBSS. The peptide solution is applied to the CAM preparation immediately and then again at 24 and 48 hrs. At 72 hours the filter paper and surrounding CAM tissue are dissected and viewed as described above.

Similar assays are performed with the organic molecules prepared as described in Example 10.

### C. Inhibition of Tumor-Induced Angiogenesis by Topical Application

### 1) Treatment with Monoclonal Antibodies

In addition to the angiogenesis assays described above where the effects of anti-αᵥβ₅ antibody and peptide antagonists were evaluated, the role of αᵥβ₅ in tumor-induced angiogenesis is also investigated. As an inducer, αᵥβ₅-negative human tissues previously grown and isolated from the CAM of a 17-day chick embryo are used. The fragments are prepared as described in Example 5C.

As described above, mAbs are separately topically applied to the tumor fragments at a concentration of 25 µg in 25 µl of HBSS and the windows are then sealed with tape. The mAbs are added again in the same fashion at 24 hours and 48 hours. At 72 hours, the tumors and surrounding CAM tissues are analyzed as described above.

As described in Example 5C, tumors are initially derived by transplanting human cell lines, which do not express integrin αᵥβ₅, onto the CAMs of 10 day old chick embryos.

In order to quantitate the effect of the mAbs on the tumor-induced angiogenesis, blood vessels entering the tumor within the focal plane of the CAM are counted under a stereo microscope by two observers in a double-blind fashion.

The synthetic peptides prepared in Example 3 and the organic molecules prepared in Example 10 are similarly topically applied to the tumor-induced angiogenic CAM assay system as described above. The effect of the peptides and organic molecules on the viability of the vessels is similarly assessed.

### D. Inhibition of Tumor-Induced Angiogenesis by Intravenous Application

### 1) Treatment with Monoclonal Antibodies

Tumor-induced blood vessels prepared above were also treated with mAbs applied by intravenous injection. CS-1 melanoma tumors were placed on the CAMs as described in Example 5C and the windows were sealed with tape and 24 hours later, 100 to 300 µg of purified mAbs were inoculated once intravenously in chick embryo blood vessels as described previously. The chick embryos were then allowed to incubate for 7 days. The extent of angiogenesis was then observed as described in above. After this time period, the tumors were resected and analyzed by their weight to determine the effect of antibody exposure on tumor growth or suppression.

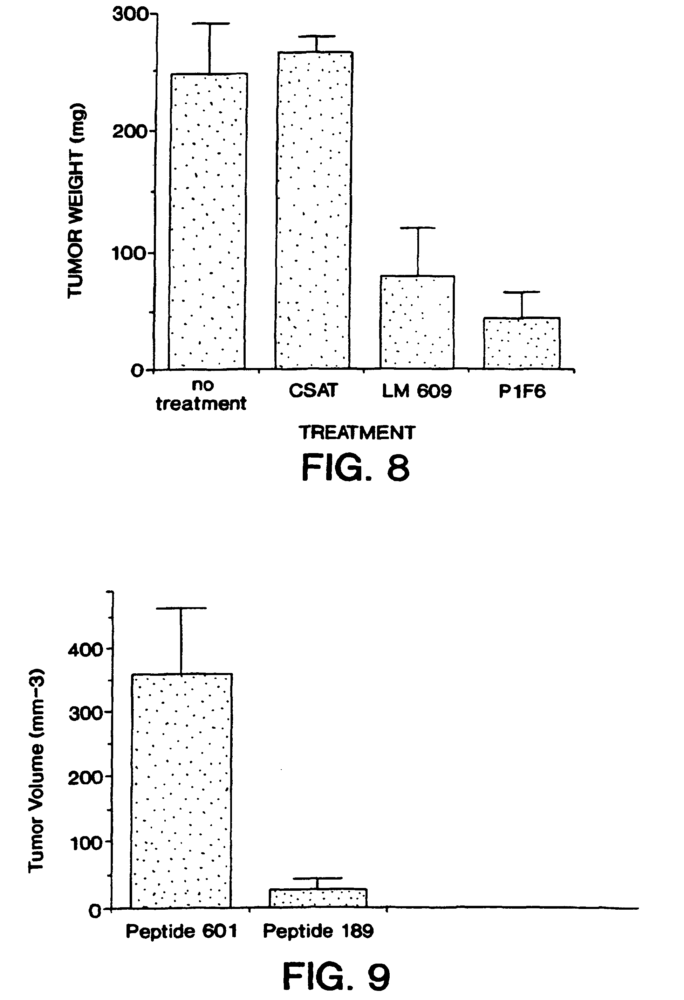

The results of treatment of CS-1 tumors with 300 µg of αᵥβ₅ specific antibody P1F6 are shown in Figure 8. The tumor weight was dramatically reduced to less than 50 mg as compared to untreated to CSAT-treated tumors. The αᵥβ₃ specific antibody, LM609, also inhibited tumor growth, however, less effective than that with P1F6. Comparable results were obtained with tumors receiving treatment with 100 µg of P1F6. Thus, PlF6 was effective at inhibiting αᵥβ₅-mediated angiogenesis in a tumor model on a CAM preparation resulting in a diminution of tumor cell mass.

### 2) Treatment with Synthetic Peptides

The effects of peptide molecules on tumor-induced vasculature in the CAM assay system is also assessed. The tumor-CAM preparation is used as described above with the exception that instead of intravenous injection of a mAb, synthetic peptides prepared as described in Example 3 are separately intravenously injected into visible blood vessels.

### 7. Identification of αᵥβ₅-Specific Antagonists Detected by a Ligand-Receptor Binding Assay

The αᵥβ₅-immunoreactive antibodies and synthetic peptides prepared respectively in Examples 1 and 3 are screened by measuring their ability to antagonize αᵥβ₅, αᵥβ₃, and α_{IIb}β₃ receptor binding activity in purified ligand-receptor binding assays. The method for these binding studies has been described by Barbas et al., Proc. Natl. Acad. Sci.. USA. 90:10003-10007 (1993), Smith et al., J. Biol. Chem., 265:11008-11013 (1990) and Pfaff et al., J. Biol. Chem., 269:20233-20238 (1994).

A method of identifying antagonists in a ligand-receptor binding assay is described in which the receptor is immobilized to a solid support and the ligand and antagonist are ' soluble. A ligand-receptor binding assay is also described in which the ligand is immobilized to a solid support and the receptor and antagonists are soluble.

Briefly, selected purified integrins are separately immobilized in Titertek microtiter wells at a coating concentration of 50 nanograms (ng) per well. The purification of the receptors used in the ligand-receptor binding assays are well known in the art and are readily obtainable with methods familiar to one of ordinary skill in the art. After incubation for 18 hours at 4C, nonspecific binding sites on the plate are blocked with 10 milligrams/milliliter (mg/ml) of bovine serum albumin (BSA) in Tris-buffered saline. For inhibition studies, various concentrations of selected antibodies or peptides are tested for the ability to block the binding of ¹²⁵I-vitronectin or other labeled ligands to the integrin receptors, αᵥβ₅, αᵥβ₃, αᵥβ₁ and α_{IIb}β₃.

Although these ligands exhibit optimal binding for a particular integrin, vitronectin for αᵥβ₅ and αᵥβ₃ and fibrinogen for α_{IIb}β₃, inhibition of binding studies using either antibodies or peptides to block the binding of vitronectin to either receptor allows for the accurate determination of the amount in micromoles (µM) of peptide necessary to half-maximally inhibit the binding of receptor to ligand. Radiolabeled ligands are used at concentrations of 1 nM and binding is challenged separately with unlabeled synthetic peptides. Following a three hour incubation, free ligand is removed by washing and bound ligand is detected by gamma counting.

Thus, the ligand-receptor assay described herein is used to screen for both circular or linearized synthetic peptides along with monoclonal antibodies and organic molecules that exhibit selective specificity for a particular integrin receptor, specifically αᵥβ₅, as used as vitronectin receptor (αᵥβ₅) antagonists in practicing this invention.

### 8. In Vivo Regression of Tumor Tissue Growth With αᵥβ₅ Antagonists As Measured by Chimeric Mouse:Human Assay

An in vivo chimeric mouse:human model was generated by replacing a portion of skin from a SCID mouse with human neonatal foreskin. The in vivo chimeric mouse:human model was prepared essentially as described in Yan, et al., J. Clin. Invest., 91:986-996 (1993). Briefly, a 2 cm² square area of skin was surgically removed from a SCID mouse (6-8 weeks of age) and replaced with a human foreskin. The mouse was anesthetized and the hair removed from a 5 cm² area on each side of the lateral abdominal region by shaving. Two circular graft beds of 2 cm² were prepared by removing the full thickness of skin down to the fascia. Full thickness human skin grafts of the same size derived from human neonatal foreskin were placed onto the wound beds and sutured into place. The graft was covered with a Band-Aid which was sutured to the skin. Micropore cloth tape was also applied to cover the wound.

After the skin graft was established, the human foreskin was inoculated with melanoma cells. The M21L human melanoma cell line was used to form the solid human tumors on the human skin grafts on the SCID mice. A single cell suspension of 2 x 10⁶ M21L was injected intradermally into the human skin graft. The mice were then observed for 2 to 4 weeks to allow growth of measurable human tumors.

After a measurable tumor was established, either 250 µg of the peptide (in a volume of 100 ul) having SEQ ID NO 9 (cyclic RGD-containing peptide Arg-Gly-Asp-D-Phe-Asn-methylated Val) or a control peptide, cyclo Arg-βAla-Asp-D-Phe-Val, were injected intraperitoneally into the mouse 3 times per week over 3 weeks. At the end of this time, the tumor was excised and analyzed by weight and histology.

The results are shown in Figure 9 where the tumor volume in mm³ is plotted on the Y-axis against the peptide treatments on the X-axis. The test peptide having SEQ ID NO 9, , labeled in the figure as peptide 189, significantly reduced the tumor volume to approximately 25 mm³ compared to control peptide (labeled as peptide 601) where the tumor volume was greater than 300 mm³.

Thus, the blocking of the αᵥβ₅ receptor by the intravenous application of αᵥβ₅ antagonist peptide 189 resulted in a regression of a melanoma tumor in this model system in the same manner as the CAM and rabbit eye model systems as described previously.

The SCID/human chimeric model above is also used for assessing the effectiveness of other αᵥβ₅ antagonists of this invention, namely antibodies and organic molecules, the latter of which are prepared as described in Example 10.

### 9. Preparation of a Murine Mouse Model for αᵥβ₅-Mediated Retinal Angiogenesis and Inhibition Thereof with αᵥβ₅ Antagonists

Based on the observation in Example 2C of αᵥβ₃ and αᵥβ₅ expression in retinal neovascular tissue, a novel mouse model was used to study the effects of systemically administered cyclic peptide antagonists of both integrins on retinal angiogenesis. Newborn mice develop retinal vessels during the first two weeks postnatally during which time the superficial retinal vasculature forms a rich, highly branched network of vessels that originate at the optic nerve head and radiate peripherally to cover the retinal surface in a manner similar to that observed in other mammals and humans (Jiang et al., Glia, 15:1-10 (1995).

For the model, newborn mice were injected subcutaneously twice daily for four days starting from day 0 with the cyclic peptide RGDfV (SEQ ID NO 4) (also referred to as peptide 203) or the control peptide RADfV (SEQ ID NO 5). On postnatal day five, globes were removed and fixed in 4.0% paraformaldehyde (PFA) at room temperature.

To quantitate mouse retinal angiogenesis, the distance from the optic nerve head to the most distal point of a single vessel selected in each of six equal sectors around a twelve hour clock was measured. The mean distance was calculated and averaged with similar data obtained from an entire litter. To measure the total volume of retinal blood vessels, the entire specimen was scanned in 2.0 µm optical sections and stored digitally. The "seed" function in Bio-Rad's Lasersharp software was then used to threshold and count cubic pixels in each section. A macro was written to sum the volume of all sections and determine the value for all vascular structures.

With the direct measurement of vessel growth in two dimensions from photographs, systemically administered peptide antagonist 203 inhibited retinal vasculogenesis, relative to control peptide, by 44% (N=9, p<.0000001, paired t-test). No statistical difference was seen between untreated newborn mice and five-day old mice receiving peptide 203, thus the peptide effectively inhibits vasculogenesis. In addition, no statistical difference was seen between untreated five-day old mice and the same aged mice receiving control peptide. Thus, inhibition of retinal vasculogenesis in RGDfV-treated newborn mice when compared to untreated counterparts is effectively 100%.

Using a more quantitative analysis taking the three dimensional nature of vessel growth, a 78% reduction in the retinal vascular volume in the peptide 203-treated anaimals compared to the controls was seen. The mean volume of vessels on postnatal day five in 203-treated animals was 3.6 X 10⁶ µm³ and in control-treated animal was 15.7 X 10⁶ µm³. The volume occupied by retinal blood vessels in untreated newborn mice was indistinguishable from the five-day old 203-treated animals.

The results obtained above showed that the antagonists specifically blocked new blood vessel formation with no effect on established vessels. The results indicate that the pathology of retinal neovascular disease is distinct from that seen with subretinal neovascular disease and that antagonists of αᵥβ₅ are effective for treating patients with blinding eye disease associated with angiogenesis.

The foregoing written specification is considered to be sufficient to enable one skilled in the art to practice the invention. Indeed, various modifications of the invention in addition to those shown and described herein will become apparent to those skilled in the art from the foregoing description and fall within the scope of the invention as defined by the appended claims.

## Claims

1. The use of an αᵥβ₅ antagonist in the manufacture of a medicament for inhibiting αᵥβ₅-mediated angiogenesis in an αᵥβ₅-containing tissue, wherein said angiogenesis is present in a patient having a corneal neovascular disorder selected from the group of disorders consisting of corneal transplantation, herpetic keratitis, luetic keratitis, pterygium and neovascular pannus associated with contact lens use, and wherein said αᵥβ₅ antagonist is an RGD-containing polypeptide.

2. The use of an αᵥβ₅ antagonist as claimed in claim 1, wherein said polypeptide is selected from the group consisting of cyclo(Arg-Gly-Asp-D-Phe-Val) (SEQ ID NO 4), cyclo(Gly-D-Arg-Gly-Asp-Phe-Val) (SEQ ID NO 6), cyclo(Arg-Gly-Asp-Phe-D-Val) (SEQ ID NO 7), Tyr-Thr-Ala-Glu-Cys-Lys-Pro-Gln-Val-Thr-Arg-Gly-Asp-Val-Phe (SEQ ID NO 8) and a salt thereof.

3. The use of an αᵥβ₅ antagonist as claimed in claim 2, wherein said salt is hydrochloride.

4. The use of an αᵥβ₅ antagonist as claimed in claim 1, wherein said αᵥβ₅ antagonist is present in an angiogenesis inhibiting amount from 2 µM to 5 mM.

5. The use of an αᵥβ₅ antagonist as claimed in claim 1, wherein said medicament is for intraocular administration.

## Patentansprüche

1. Verwendung eines αᵥβ₅-Antagonisten zum Herstellen eines Medikaments zum Hemmen einer αᵥβ₅-vermittelten Angiogenese in einem αᵥβ₅-enthaltenden Gewebe, wobei die Angiogenese bei einem Patienten vorliegt, der an einer neovaskulären Störung der Hornhaut des Auges leidet, die aus der Gruppe von Störungen ausgewählt ist, die aus Homhauttransplantation, Herpes-Keratitis, Lues-Keratitis, Pterygium und neovaskulärem Pannus, assoziiert mit dem Tragen von Kontaktlinsen, besteht, und wobei der αᵥβ₅-Antagonist ein RGD-enthaltendes Polypeptid ist.

2. Verwendung eines αᵥβ₅-Antagonisten nach Anspruch 1, wobei das Polypeptid aus der Gruppe ausgewählt ist, die aus Cyclo-(Arg-Gly-Asp-D-Phe-Val) (SEQ ID NO: 4), Cyclo-(Gly-D-Arg-Gly-Asp-Phe-Val) (SEQ ID NO: 6), Cyclo-(Arg-Gly-Asp-Phe-D-Val) (SEQ ID NO: 7), Tyr-Thr-Ala-Glu-Cys-Lys-Pro-Gln-Val-Thr-Arg-Gly-Asp-Val-Phe (SEQ ID NO: 8) und einem Salz davon besteht.

3. Verwendung eines αᵥβ₅-Antagonisten nach Anspruch 2, wobei das Salz ein Hydrochlorid ist.

4. Verwendung eines αᵥβ₅-Antagonisten nach Anspruch 1, wobei der αᵥβ₅-Antagonist in einer die Angiogenese hemmenden Menge von 2 µM bis 5 mM vorliegt.

5. Verwendung eines αᵥβ₅-Antagonisten nach Anspruch 1, wobei das Medikament für die intraokulare Verabreichung vorgesehen ist.

## Revendications

1. L'utilisation d'un antagoniste d'αᵥβ₅ dans la fabrication d'un médicament pour inhiber une angiogénèse à médiation d'αᵥβ₅ dans un tissu contenant αᵥβ₅, dans laquelle ladite angiogénèse est présente chez un patient ayant un désordre néovasculaire cornéen sélectionné dans le groupe des désordres se composant de transplantation cornéenne, kératite herpétique, kératique syphilitique, ptérygion et pannus néovasculaire associé à une utilisation de lentilles de contact, et dans lequel ledit antagoniste d'αᵥβ₅ est un polypeptide contenant RGD.

2. L'utilisation d'un antoganiste d'αᵥβ₅ telle que revendiquée à la revendication 1, dans lequel ledit polypeptide est sélectionné dans le groupe se composant de cyclo(Arg-Gly-Asp-D-Phe-Val) (ID SEQ NO 4), cyclo(Gly-D-Arg-Gly-Asp-Phe-Val) (ID SEQ NO 6), cyclo(Arg-Gly-Asp-Phe-D-Val) (ID SEQ NO 7), Tyr-Thr-Ala-Glu-Cys-Lys-Pro-Gln-Val-Thr-Arg-Gly-Asp-Val-Phe (ID SEQ NO 8) et un sel de ceux-ci.

3. L'utilisation d'un antagoniste d'αᵥβ₅ telle que revendiquée à la revendication 5, dans lequel ledit sel est un hydrochlorure.

4. Utilisation d'un antogoniste d'αᵥβ₅ telle que revendiquée à la revendication 1, dans lequel ledit antagoniste d'αᵥβ₅ est présent dans une quantité d'inhibition d'angiogénèse de 2 µM à 5 mM.

5. L'utilisation d'un antagoniste d'αᵥβ₅ telle que revendiquée à la revendication 1, dans lequel ledit médicament est pour une administration intraoculaire.
